# FASCICULE DE BREVET EUROPEEN

(11) **EP 1 878 723 B1**
(45) Date de publication et mention de la délivrance du brevet: **26.05.2010**
(21) Numéro de dépôt: 06014422.7
(22) Date de dépôt: 11.07.2006
(51) Int. Cl.: C07D 231/14, C07D 409/12, C07D 405/12, C07D 413/12, C07D 403/06, C07D 403/04, C07D 231/12, C07D 231/18, A61K 31/4155, A61P 25/28

(54) **Dérivés de N-[(1,5-diphényl-1H-pyrazol-3-yl)méthyl]sulfonamide antagonistes des récepteurs CB1 des cannabinoïdes**
N-[(1,5-diphenyl-1H-pyrazol-3-yl)methyl]sulfonamid-Derivate Antagonisten der CB1 Rezeptoren der Cannabinoide
N-[(1,5-diphenyl-1H-pyrazol-3-yl)methyl]sulfonamide derivatives antagonists of the CB1 cannabinoids receptors

(43) Date de publication de la demande: 16.01.2008
(73) Titulaire: Sanofi-Aventis, 75013 Paris (FR)
(72) Inventeur: Barth, Francis, 34680 Saint Georges d'Orques (FR); Congy, Christian, 34980 Saint Gely du Fesc (FR); Martinez, Serge, 34000 Montpellier (FR); Pointeau, Philippe, 34980 Saint Clement de Riviere (FR); Rinaldi-Carmona, Murielle, 34680 Saint Georges d'Orques (FR)
(74) Mandataire: Kugel, Dominique

(56) Documents cités:
- EP-A1- 0 576 357
- WO-A-2004/052864
- WO-A-2005/073197
- WO-A2-2005/000820
- PERTWEE R G: "PHARMACOLOGY OF CANNABINOID RECEPTOR LIGANDS" CURRENT MEDICINAL CHEMISTRY, BENTHAM SCIENCE PUBLISHERS BV, BE, vol. 6, no. 8, 1999, pages 635-664, XP009024021 ISSN: 0929-8673

## Description

La présente invention a pour objet des dérivés de N-[(1,5-diphényl-1*H*-pyrazol-3-yl)méthyl]sulfonamide, leur préparation et leur application en thérapeutique.

Des dérivés de diphénylpyrazole, présentant une affinité pour les récepteurs CB₁ des cannabinoïdes ont été décrits notamment dans les brevets EP 0 576 357, EP 0 656 354 et US 5 624 941.

La demande de brevet internationale WO2005/073 197décrit des dérivés de N-[1,5-diphényl-4-méthyl-1*H*-pyrazole-3-yl)méthyl]sulfonamide, antagonistes des récepteurs aux cannabinoïdes CB₁.

On a maintenant trouvé des nouveaux dérivés de N-[(1,5-diphényl-1*H*-pyrazol-3-yl)méthyl]sulfonamide qui possèdent des propriétés antagonistes des récepteurs CB₁ des cannabinoïdes, localisés au niveau central et/ou périphérique.

La présente invention a pour objet des composés répondant à la formule (I) : 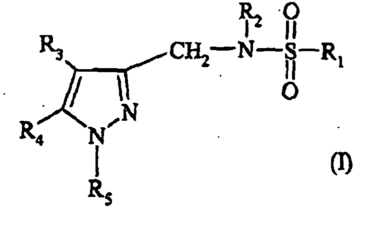 dans laquelle :
- R₁ représente
   . un phényle non substitué ou substitué par un ou plusieurs substituants choisis indépendamment parmi un atome d'halogène, un (C₁-C₄)alkyle, un (C₁-C₄)alcoxy, un cyano, un groupe trifluorométhyle, un groupe trifluorométhoxy, un groupe S(O)ₙAlk, un groupe (C₁-C₄)alkylcarbonyle, un phényle ;
   . un benzyle non substitué ou substitué par un ou plusieurs substituants choisis indépendamment parmi un atome d'halogène, un (C₁-C₄)alkyle, un (C₁-C₄)alcoxy ; un groupe trifluorométhyle ;
   . un radical thiényle, furyle, oxazolyle, thiazolyle, imidazolyle, ledit radical étant non substitué ou substitué par un ou plusieurs substituants choisis indépendamment parmi un atome d'halogène, un (C₁-C₄)alkyle, un groupe trifluorométhyle ;
   . un naphtyle non substitué ou substitué par un ou plusieurs substituants choisis indépendamment parmi un (C₁-C₄)alkyle, un di(C₁-C₄)akylamino ;
   . un 2,3-dihydrobenzofuran-yle non substitué ou substitué une ou plusieurs fois par un groupe (C₁-C₄)alkyle ;
- R₂ représente un atome d'hydrogène ou un (C₁-C₄)alkyle ;
- R₃ représente un cyano, un hydroxyle, un (C₁-C₄)alcoxy, un cyanométhyle, un hydroxyméthyle, un (C₁-C₄)alcoxyméthyle, un fluorométhyle, un tétrazolylméthyle, un N-(méthyl)tétrazolylméthyle, un tétrazolyle, un N-(méthyl)tétrazolyle, un groupe CONR₆R₇, un groupe CH₂S(O)ₙAlk, un groupe COOR₈ ;
- R₄ et R₅ représentent chacun indépendamment un phényle non substitué ou substitué par un ou plusieurs substituants choisis indépendamment parmi un atome d'halogène, un (C₁-C₇)alkyle, un (C₁-C₄)alcoxy, un groupe trifluorométhyle ou un groupe S(O)ₙAlk ;
- R₆ et R₇ représentent chacun indépendamment un atome d'hydrogène ou un (C₁-C₄)alkyle ou R₆ et R₇ ensemble avec l'atome d'azote auquel ils sont liés constituent un radical hétérocyclique choisi parmi pyrrolidinyle, pipéridinyle, morpholinyle, pipérazinyle non substitué ou substitué une ou plusieurs fois par un (C₁-C₄)alkyle ;
- R₈ représente un (C₁-C₄)alkyle ;
- n représente 0, 1 ou 2 ;
- Alk représente un (C₁-C₄)alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₄)alkyle ou respectivement (C₁-C₇)alkyle ou (C₁-C₁₂)alkyle, on entend un radical alkyle linéaire ou ramifié de un à quatre atomes de carbone ou respectivement de un à sept ou de un à douze atomes de carbone, tel que le radical méthyle, éthyle, propyle, isopropyle, butyle, isobutyle, *sec*-butyle, *tert*-butyle, pentyle, isopentyle, hexyle, isohexyle, heptyle,...

Par (C₁-C₄)alkyle substitué une ou plusieurs fois par un atome de fluor, on entend en particulier les groupes difluorométhyle, trifluorométhyle, difluorométhyle, trifluoroéthyle.

Par (C₁-C₄)alcoxy on entend un radical alcoxy linéaire ou ramifié de un à quatre atomes de carbone ou respectivement de un à cinq atomes de carbone, tel que le radical méthoxy, éthoxy, propoxy, isopropoxy, butoxy, *sec*-butoxy, *tert*-butoxy.

Par (C₃-C₇)cycloalkyle on entend un groupe alkyle cyclique de 3 à 7 atomes de carbone, tel que le groupe cyclopropyle, cyclobutyle, cyclopentyle, cyclohexyle, cycloheptyle.

Par tétrazolyle on entend un radical tétrazol-1-yle, tétrazol-2-yle ou tétrazol-5-yle.

Parmi les composés de formule (I) selon l'invention on distingue :
- les composés de formule I_{A} dans laquelle R₃ est un cyano ;
- les composés de formule I_{B} dans laquelle R₃ est un hydroxyle ;
- les composés de formule I_{C} dans laquelle R₃ est un (C₁-C₄)alcoxy ;
- les composés de formule I_{D} dans laquelle R₃ est un cyanométhyle ;
- les composés de formule I_{E} dans laquelle R₃ est un hydroxyméthyle ;
- les composés de formule I_{F} dans laquelle R₃ est un (C₁-C₄)alcoxyméthyle
- les composés de formule I_{G} dans laquelle R₃ est un fluorométhyle ;
- les composés de formule I_{H} dans laquelle R₃ est un groupe CH₂S(O)ₙAlk ;
- les composés de formule I_{I} dans laquelle R₃ est un groupe CONR₆R₇ ;
- les composés de formule I_{J} dans laquelle R₃ est un COOR₈ ;
- les composés de formule I_{K} dans laquelle R₃ est un tétrazol-5-yle ;
- les composés de formule I_{L} dans laquelle R₃ est un N-(méthyl)tétrazol-5-yle ;
- les composés de formule I_{M} dans laquelle R₃ est un tétrazol-5-ylméthyle ;
- les composés de formule I_{N} dans laquelle R₃ est un N-(méthyl) tétrazol-5-ylméthyle ;
- les composés de formule I_{O} dans laquelle R₃ est un tétrazol-1-ylméthyle ou un tétrazol-2-ylméthyle ;
les groupes Alk, R₆, R₇ et R₈ étant tels que définis ci-dessous pour (I).

Parmi les composés de formule (I), objets de l'invention, on préfère les composés dans lesquels :
- R₁ représente :
   . un radical phényle, benzyle, dans lequel chaque groupe phényle est non substitué ou substitué une ou plusieurs fois par des substituants choisis indépendamment parmi un atome d'halogène, un (C₁-C₄)alkyle, un (C₁-C₄)alcoxy, un trifluorométhyle ;
   . un radical furyle non substitué ou substitué par un ou plusieurs substituants choisis indépendamment parmi un atome d'halogène, un (C₁-C₄)alkyle, un groupe trifluorométhyle ;
- R₂ représente un atome d'hydrogène ;
- R₃ représente un cyano, un hydroxyle, un (C₁-C₄)alcoxy, un hydroxyméthyle, un (C₁-C₄)alcoxyméthyle, un groupe CONR₆R₇, un groupe COOR₈, un tétrazol-1-yl méthyle ou un tétrazol-2-ylméthyle, les groupes R₆, R₇, R₈ étant tels que définis pour (I) ;
- R₄ représente un 4-chlorophényle, un 4-méthoxyphényle ;
- R₅ représente un 2-chlorophényle, un 2-bromophényle ou un 2,4-dichlorophényle ; à l'état de base ou de sel d'addition à des acides ainsi qu'à l'état d'hydrates ou de solvats.

Plus particulièrement, on préfère les composés de formule (I) dans laquelle :
- R1 représente un groupe 3-chlorophényle, 3-fluorophényle, 3,6-difluorophényle, 2,6-difluorophényle, 3-méthoxyphényle, 3-trifluorométhylphényle, 3-trifluorométhoxyphényle, un benzyle, un 4-trifluorométhylbenzyle ou un 2-trifluorométhyl-4-méthylfuryle ;
- R₂ représente un atome d'hydrogène ;
- R₃ représente un groupe cyano, méthoxy ou diméthylaminocarbonyle ;
- R₄ représente un 4-chlorophényle, un 4-méthoxyphényle ;
- R₅ représente un 2,4-dichlorophényle ou un 2-chlorophényle ;
à l'état de base ou de sel d'addition à des acides ainsi qu'à l'état d'hydrates ou de solvats.

Tout particulièrement, les composés suivants sont préférés :
- ex 6 : SSR 136477 le N-{[5-(4-chlorophényl)-4-cyano-1-(2,4-dichlorophényl)-1*H-*pyrazol-3-yl]méthyl}-3-cyanobenzènesulfonamide,
- le N-{[5-(4-chlorophényl)-4-cyano-1-(2,4-dichlorophényl)-1*H*-pyrazol-3-yl]méthyl}-3-trifluorobenzènesulfonamide,
- le N-{[5-(4-chlorophényl)-4-cyano-1-(2,4-dichlorophényl)-1*H*-pyrazol-3-yl]méthyl}-2-trifluorométhoxybenzcnesuHbnamide,
- le N-{[5-(4-chlorophényl)-4-cyano-1-(2,4-dichlorophényl)-1*H*-pxazo1-3-yl]méthyl}-3-méthoxybenzènesulfonamide,
- le N-{[5-(4-chlorophényl)-4-cyano-1-(2,4-dichlorophényl)-1*H-*pyrazol-3-yl]méthyl}
- 3-chlorobenzènesulfonamide,
- le N-{[5-(4-chlorophényl)-4-cyano-1-(2-chlorophényl)-1*H*-pyrazol-3-yl]méthyl}-3-fluorobenzénesulfonamide,
- le N-{[5-(4-chlorophényl)-4-cyano-1-(2-chlorophényl)-1*H*-pyrazol-3-yl]méthyl}-2-fluorobenzènesulfonamide,
- le N-{[5-(4-chlorophényl)-4-cyano-1-(2-chlorophényl)-1*H*-pyrazol-3-yl]méthyl}-2-trifluorométhoxybenzènesulfonamide,
- le N-{[5-(4-méthoxyphényl)-4-cyano-1-(2,4-dichlorophényl)-1*H*-pyrazol-3-yl]méthyl}-3,5-difluorobenzènesulfonamide,
- le N-{[5-(4-propanesulfonyloxyphényl)-4-cyano-1-(2,4-dichlorophényl)-1*H*-pyrazol-3-yl]méthyl}-3,5-difluorobenzènesulfonamide,
- le N-{[5-(4-chlorophényl)-4-méthoxy-1-(2,4-dichlorophényl)-1*H*-pyrazol-3-yl]méthyl}-3-chlorobenzènesulfonamide,
- le 5-(4-chlorophényl)-3-({[(3-chlorophényl)sulfonyl]amino}méthyl)-1-(2,4-dichlorophényl)-*N*-méthyl-1*H*-pyrazole-4-carboxamide,
- le N-{[5-(4-chlorophényl)-4-cyano-1-(2,4-dichlorophényl)-1*H*-pyrazol-3-yl]méthyl}-1-phénylméthanesulfonamide,
- le N-{[5-(4-chlorophényl)-4-cyano-1-(2,4-dichlorophényl)-1*H*-pyrazol-3-yl]méthyl}-5-méthyl-2-(trifluorométhyl)furane-3-sulfonamide ;
à l'état de base ou de sel d'addition à des acides ainsi qu'à l'état d'hydrates ou de solvats.

Conformément à l'invention, on peut préparer les composés de formule (I) selon un procédé qui est caractérisé en ce que : on fait réagir, en présence d'une base et dans un solvant, un composé de formule : 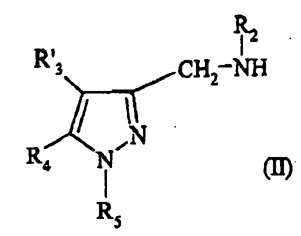 dans laquelle R₂, R₄, R₅ sont tels que définis pour un composé de formule (I) et R'₃ représente R₃ ou un précurseur de R₃, avec un halogénure de sulfonyle de formule HalSO₂R₁, dans laquelle R₁ est tel que défini pour un composé de formule (I) et Hal représente un atome d'halogène.

Le cas échéant, on transforme le composé obtenu de formule : 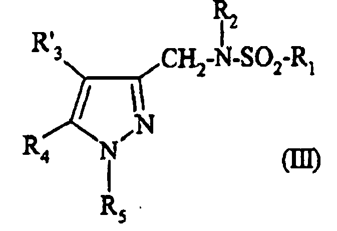 dans laquelle R'₃ est R₃ ou est un précurseur de R₃, en un composé de formule (I).

Eventuellement, on transforme le composé de formule (I) en l'un de ses sels d'addition à un acide.

Les composés de formule (I) dans laquelle R₂ représente un (C₁-C₃)alkyle peuvent aussi être préparés à partir des composés correspondant de formule (I) dans laquelle R₂ représente un atome d'hydrogène par une méthode choisie parmi les méthodes connues de l'homme de l'art. Parmi celles-ci on peut citer l'alkylation par un halogénure d'alkyle, l'amination réductrice par un aldéhyde en milieu réducteur, ou encore l'acylation par un chlorure d'acyle, suivi d'une réduction.

Par précurseur de R₃, on entend un groupe que l'on peut facilement convertir en un substituant R₃ selon l'invention.

Selon le procédé de la présente invention, la réaction de couplage d'un composé de formule (II) avec un halogénure de sulfonyle s'effectue en présence d'une base telle que la triéthylamine ou la diisopropyléthylamine, dans un solvant tel que le dichlorométhane ou le tétrahydrofurane, et à une température comprise entre la température ambiante et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Dans le cas des composés de formule (I_{E}), le groupe R'₃ précurseur de R₃ représente un groupe (tétrahydropyranyloxyméthyle) -CH₂OTHP.

Le composé de formule : 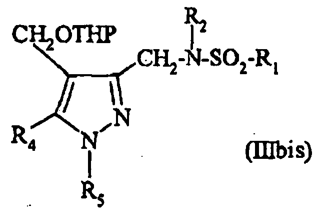 obtenu par le procédé selon la présente invention est ensuite hydrolysé en milieu acide pour donner un composé de formule (I_{E}) dans laquelle R₃ est un hydroxyméthyle.

Les composés de formule (I_{C} ; R₃ = (C₁-C₄)alcoxy) permettent d'obtenir les composés de formule (I_{B} ; R₃ = OH) par déalkylation, par exemple par action de BBr₃ ou HBr.

Par des traitements appropriés, connus de l'homme de l'art, on transforme le groupe hydroxyméthyle pour obtenir les composés de formule (I_{I}) à (I_{M}) dans laquelle R₃ a différentes valeurs.

Les composés de formule (I_{K} ; R₃ = tétrazolyle) et (I_{M} ; R₃ = tétrazolylméthyle) peuvent également être préparés à partir des composés de formule (I_{A}; R₃ = CN) et (I_{D} ; R₃ = CH₂CN).

La préparation des intermédiaires de formule (II) peut être effectuée de différentes manières selon la valeur du substituant R₃.

Lorsque R₃ représente un cyano, on procède selon la schéma réactionnel suivant :

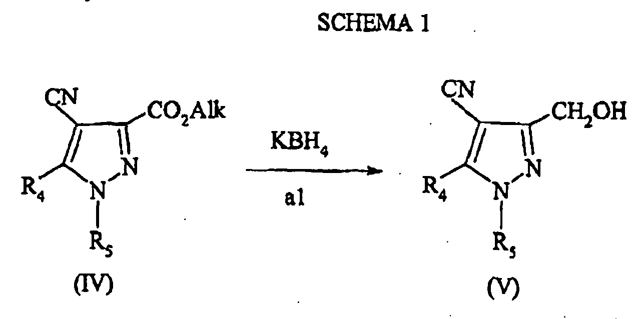

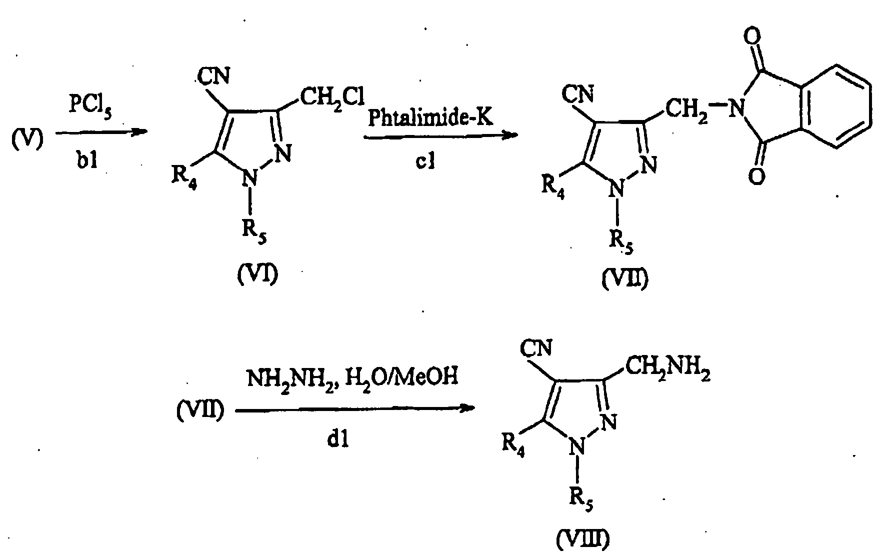

Le composé de formule (IV) est préparé selon le procédé décrit dans la demande de brevet WO 2005/000820. A l'étape a1, on effectue la réduction sélective de la fonction ester par KBH₄ ou LiAlH₄, A l'étape b1, le composé de formule (V) est halogéné, par exemple par PCl₅. Le composé de formule (VI) ainsi obtenu est ensuite traité par le phtalimide de potassium, puis, à l'étape d1, on fait agir l'hydrate d'hydrazine dans un alcool pour obtenir le composé de formule (VIII) qui correspond à l'intermédiaire de formule (II) dans laquelle le substituant R₃ est un cyano.

Lorsque R₃ représente un (C₁-C₄)alcoxy, on procède selon le Schéma réactionnel suivant pour préparer l'intermédiaire de formule (II) correspondant.

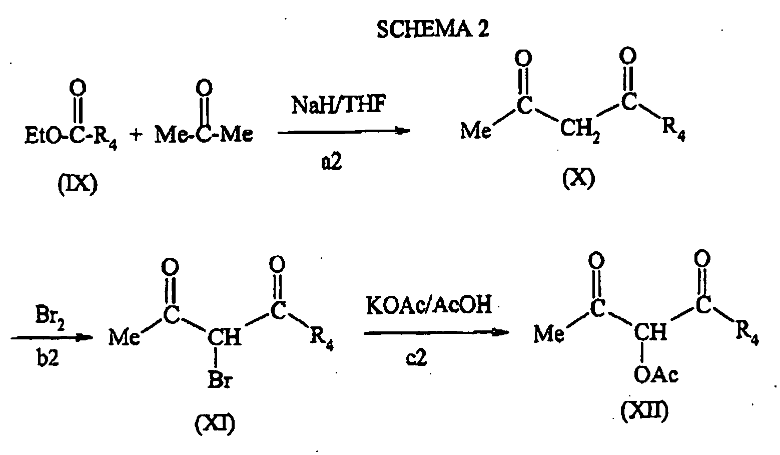

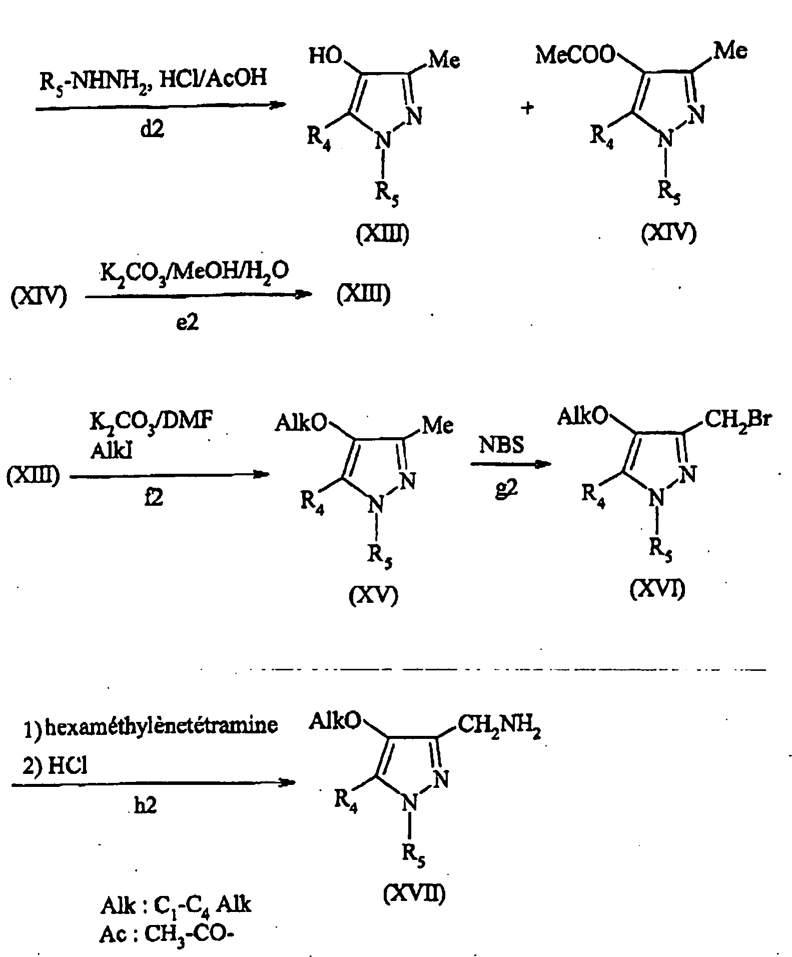

A l'étape a2, on prépare le dérivé arylbutane-1,3-dione (X) par action de l'acétone et d'un hydrure tel que l'hydrure de sodium dans le THF sur l'ester R₄CO₂Et. On obtient le composé de formule (XI) par bromation, puis on acétyle à l'étape c2 pour former le composé de formule (XII). A l'étape d2, l'action du chlorhydrate d'arylhydrazine conduit au mélange des composés de formule (XIII) et (XIV). Le composé de formule (XIV) est hydrolysé pour se transformer en composé de formule (XIII). On traite ensuite le composé de formule (XIII) par un halogénure de (C₁-C₄)alkyle de formule Alkl pour former le composé de formule (XV). A l'étape g2, on fait agir le N-bromosuccinimide (NBS) pour préparer le composé de formule (XVI) puis à l'étape h2, on fait agir l'hexaméthylènetétramine pour former le composé de formule (XVII) qui correspond à l'intermédiaire de formule (II) dans laquelle R₃ est un (C₁-C₄)alcoxy.

A l'étape g2, la bromation peut conduire également, à un composé dibromé de formule (XVIII). A partir de ce composé, on peut préparer un composé de formule (XVII) en procédant selon le Schéma réactionnel ci-après :

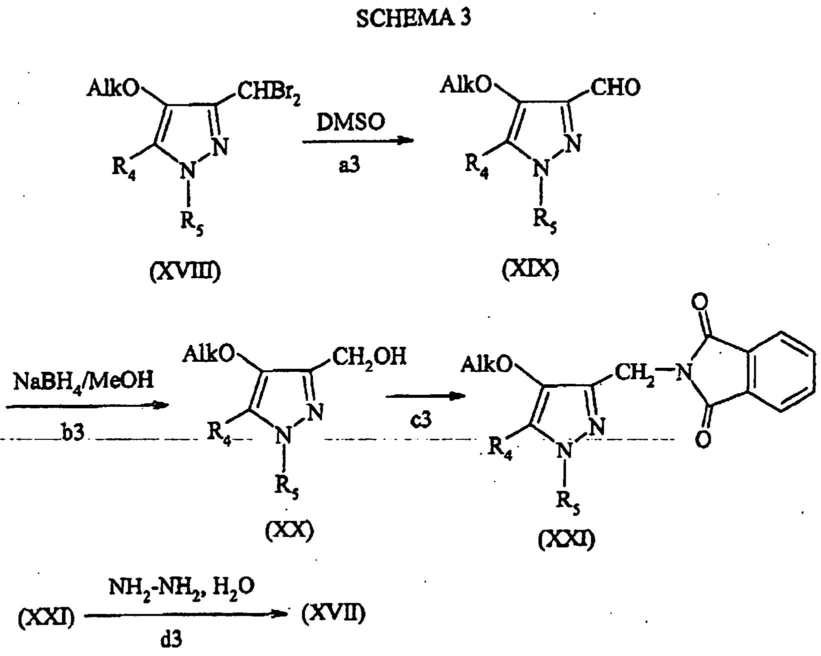

A l'étape a3, on traite le dérivé dibromé de formule (XVIII) par le DMSO pour obtenir l'aldehyde de formule (XIX) puis, à l'étape b3, on réduit par un hydrure métallique, par exemple le borohydrure de sodium ou de potassium, pour former le composé de formule (XX). A l'étape c3, l'addition du phtalimide est réalisée en présence de diéthylazodicarboxylate (DEAD). Le composé ainsi obtenu de formule (XXI) est ensuite traité par l'hydrate d'hydrazine pour former le composé de formule (XVII).

Lorsque R₃ représente un groupe cyanométhyle, (C₁-C₄)alcoxyméthyle, fluorométhyle ou (C₁-C₄)alkylthiométhyle, les intermédiaires de formule (II) correspondants sont préparés selon le Schéma réactionnel ci-après.

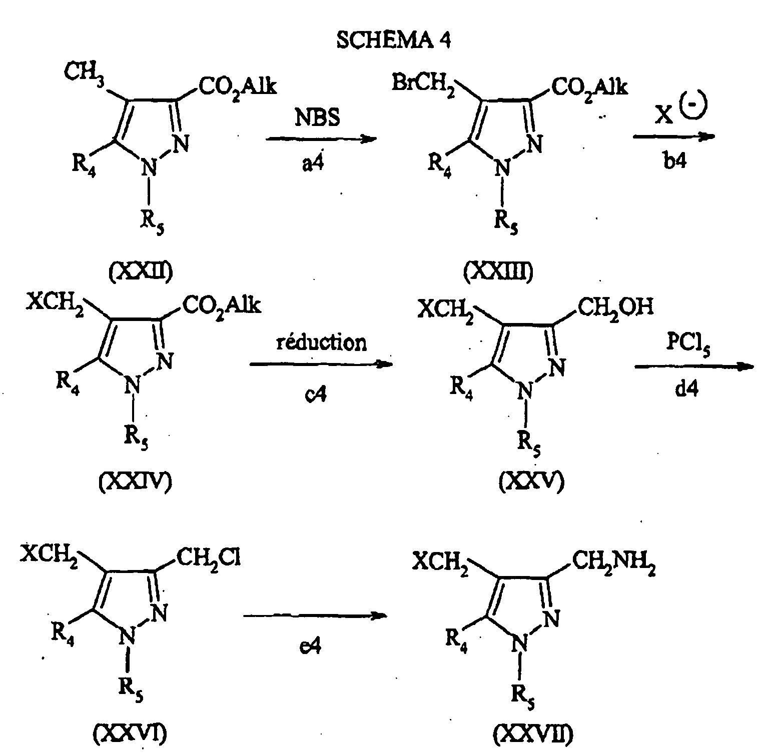

Les composés de formule (XXII) sont décrits dans le brevet EP 576 357.

A l'étape a4, le groupement méthyle du composé de formule (XXII) est bromé, par action de la NBS.

A l'étape b4, on substitue le brome par un groupe nucléophile X choisi parmi un atome de fluor, un cyano, un (C₁-C₄)alcoxy, un (C₁-C₄)alkylthio. On réduit ensuite la fonction ester par un agent réducteur tel que LiAlH₄ ou KBH₄ pour former le composé de formule (XXV). A l'étape d4, ce composé est traité par un agent tel que PCl₅ pour donner le composé de formule (XXVI). A l'étape e4, on fait agir successivement le phtalimide de potassium et l'hydrazine ou bien l'hexaméthylène tétramine et l'acide chlorhydrique pour former le composé de formule (XXVIII) qui correspond à un composé de formule (II) dans laquelle R₂ est CH₂X.

Lorsque R₃ représente un groupe CH₂SOAlk ou CH₂SO₂Alk les intermédiaires de formule (II) correspondant sont préparés à partir de l'intermédiaire de formule (XXTV) dans laquelle X = SAlk par une réaction d'oxydation pour obtenir les intermédiaires de formule (XXIV) dans laquelle X = SOAlk ou SO₂Alk. Le réactif d'oxydation peut être l'acide métachloroperbenzoïque ou bien l'eau oxygénée Les intermédiaires de formule (XXIV) sont ensuite traités comme dans le Schéma 4.

Pour préparer l'intermédiaire de formule (II) dans laquelle R'₃ est un précurseur de l'hydroxyméthyle à savoir un groupe tétrahydropyranyloxyméthyle (CH₂OTHP) on procède selon le Schéma réactionnel suivant.

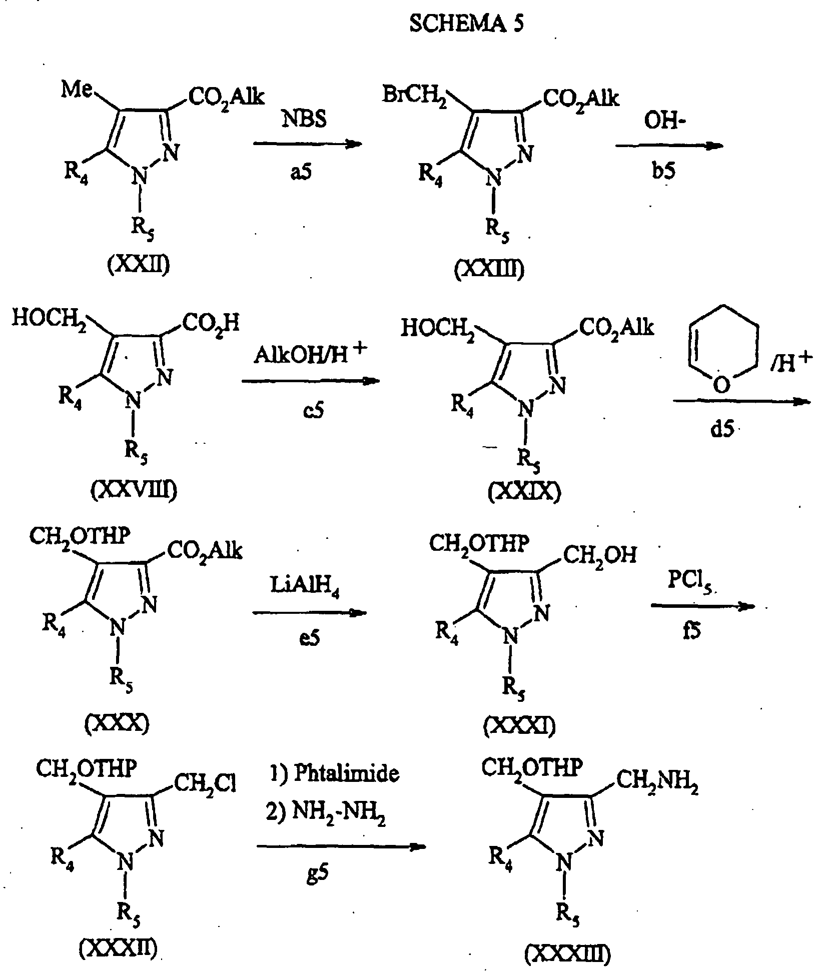

L'étape a5 est réalisée comme décrit ci-dessus pour l'étape a4. A l'étape b5, la substitution du brome par le groupe OH et l'hydrolyse de l'ester en milieu basique conduisent au composé de formule (XXVIII). Ce composé est estérifié en milieu acide pour former le composé de formule (XXIX) puis à l'étape d5, on protège le groupement hydroxyle par un groupe tel que tétrahydropyranyle ou *tert*butoxyméthyle. On conduit alors les étapes e5, f5 et g5 tel que décrit ci-dessus pour le Schéma 1, afin de préparer le composé de formule (XXXIII) qui correspond à un intermédiaire de formule (II) dans laquelle R'₃ est un groupe tétrahydropyranyloxyméthyle.

En appliquant le procédé selon l'invention au composé de formule (XXXIII), on prépare un composé de formule :

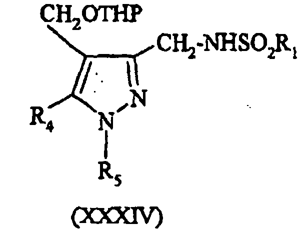

Pour obtenir un composé selon l'invention de formule (I) dans laquelle R₃ et un hydroxyméthyle, on traite le composé de formule (XXXIII) en milieu acide, dans un solvant alcoolique tel que le méthanol.

A partir du composé ainsi obtenu de formule : 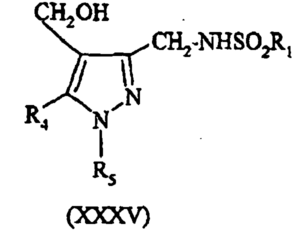 on prépare, par oxydation, un dérivé d'acide pyrazole carboxylique de formule :

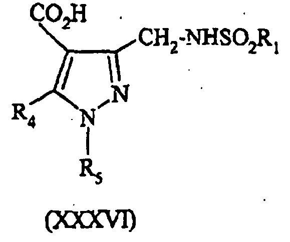

Par des méthodes connues de l'homme de l'art, on peut ensuite préparer les composés selon l'invention de formule :

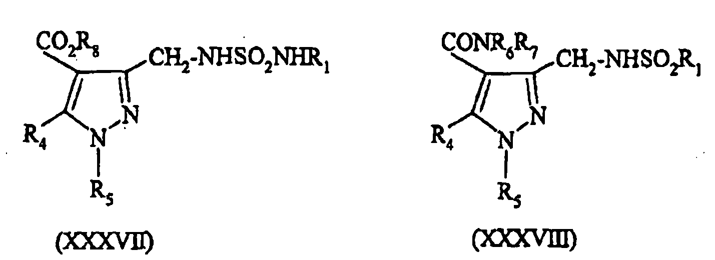

A partir d'un composé selon l'invention de formule (XXXV) ou (XXXX) dans laquelle R₃ est un hydroxyméthyle ou un cyanométhyle, on peut préparer un composé selon l'invention de formule (I) dans laquelle R₃ est un tétrazolyhnéthyle selon un des schémas réactionnels suivants :

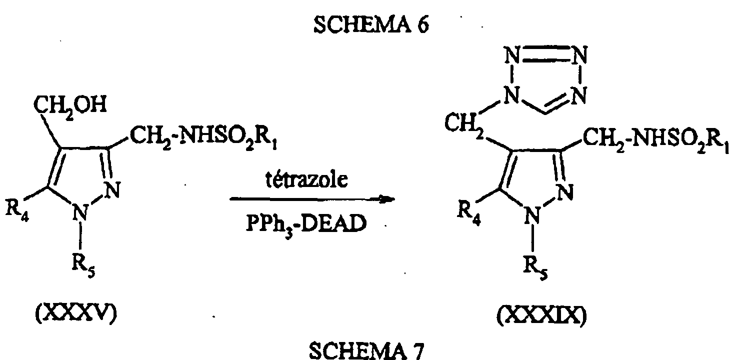

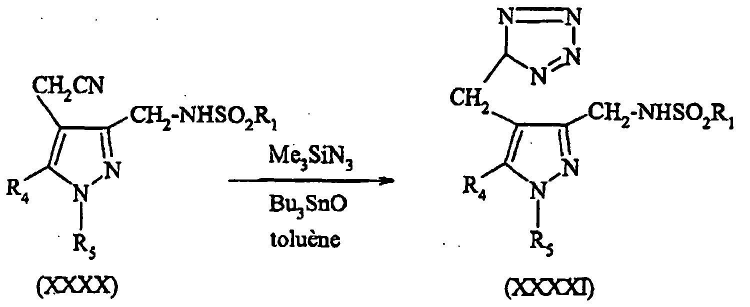

A partir d'un composé selon l'invention de formule (I) dans laquelle R₃ représente un cyano, on peut préparer un composé de formule (I) dans laquelle R₃ représente un tétrazolyle par action de l'azide de tributylétain dans un solvant tel que le xylène selon le Schéma réactionnel suivant :

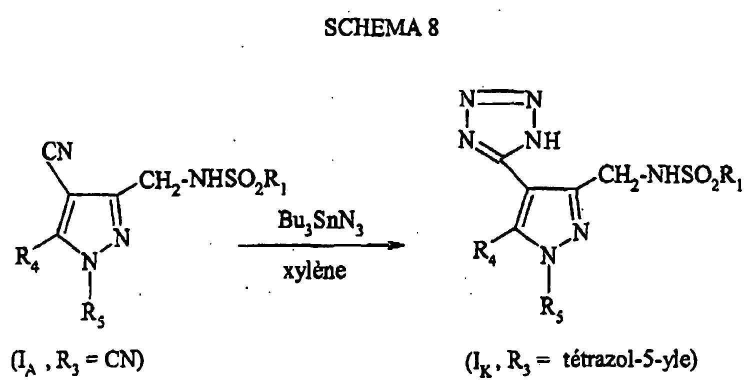

Le cas échéant, un composé selon l'invention de formule (I) dans laquelle R₃ est un N-(méthyl)tétrazolyle, ou un N-(méthyl)tétrazolylméthyle est préparé par alkylation du tétrazole par un agent alkylant.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans les TABLEAUX 1 et 2 ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
MeOH : méthanol
EtOH : éthanol
AcOH : acide acétique
DIPEA : diisopropyléthylamine
TFA : acide trifluoroacétique
Ether chlorhydrique 2N : solution 2N d'acide chlorhydrique dans 1'éther diéthylique
NBS : N-bromosuccinimide
AIBN : 2,2'-Azobis(2-méthylpropionitrile)
PPh₃ : triphénylphosphine
DEAD : diéthylazodicarboxylate
PTSOH : acide paratoluène sulfonique
BOP : hexafluorophosphate de benzotriazol-1-yloxotris(diméthylamino) phosphonium
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi, dd : doublet dédoublé.

Les composés selon l'invention sont analysés par couplage LC/UV/MS (chromatographie liquide/détection UV/spectrométrie de masse). On mesure le pic moléculaire (MH⁺) et le temps de rétention (tr) en minutes.

### Conditions A :

On utilise une colonne Symmetry C18 de 2,1 x 50 mm, 3,5 µm, à 30°C, débit 0,4 ml/minute.

L'éluant est composé comme suit :
- solvant A : 0,005 % d'acide trifluoroacétique (TPA) dans l'eau à pH 3,15 ;
- solvant B : 0,005 % de TFA dans facétonitrile.
Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nm et la détection de masse en mode ionisation chimique ESI (de l'anglais Electro Spray Ionisation) positif.

### Conditions MS2

On utilise une colonne XTERRA MS C18 de 2,1 x 30 mm, 3,5 µm, débit 0,8 ml/minute.

L'éluant est composé comme suit :
Solvant A : 0,025 % de TFA dans l'eau,
Solvant B : 0,025 % de TFA dans l'acétonitrile.
Gradient

| Temps (mn) | % A | % B |
|---|---|---|
| 0 | 100 | 0 |
| 2 | 0 | 100 |
| 2,7 | 0 | 100 |
| 2,75 | 100 | 0 |

La détection UV est effectuée par un détecteur à barette d'iode entre 210 et 400 nm et la détection de masse en mode ESI positif

### Conditions MS5

Ces conditions d'analyse LC/MS sont semblables aux conditions MS2, avec un débit de 1ml/mn.

### Préparation 1

Chlorhydrate de 3-(aminométhyl)-1-(2,4-dichlorophényl)-5-(4-méthoxyphényl)-1*H*-pyrazole-4-carbonitrile.

### A) 1-(2,4-Dichlorophényl)-3-hydroxyméthyl-5-(4-méthoxyphényl)-1H-pyrazole-4-carbonitrile.

On prépare une solution de 11,5 g de 4-cyano-1-(2,4-dichlorophényl)-5-(4-méthoxyphényl)-1*H*-pyrazole-3-carboxylate d'éthyle (préparé selon la demande de brevet WO 2005/000820) dans 150 ml de THF et on ajoute 1,8 g de KBH₄ et 1,5 g de LiCl puis on laisse sous agitation pendant une nuit à TA et on chauffe à reflux pendant 2 heures et demie. Le milieu réactionnel est refroidi à TA puis filtré et lavé par du THF. Le filtrat est évaporé à sec puis le résidu est dilué par AcOEt et lavé à l'eau. La phase organique est séchée sur Na₂SO₄, filtrée et évaporée à sec. Le résidu est trituré dans du pentane puis filtré. On obtient 10 g du composé attendu.

### B) 3-(Chlorométhyl)-1-(2,4-dichlorophényl)-5-(4-méthoxyphényl)-1H-pyrazole-4-carbonitrile.

A une solution de 10 g du composé obtenu à l'étape précédente dans 200 ml de DCM, on ajoute à 0°C 7,2 g de PCl₅ par petites portions et on laisse sous agitation 20 minutes à 0°C puis 24 heures à TA. Le milieu réactionnel est versé sur un mélange eau/glace puis on sépare la phase organique et on re-extrait la phase aqueuse au DCM. Les phases organiques sont réunies et séchées sur Na₂SO₄ puis filtrées et évaporées à sec. Le résidu est trituré dans du pentane et l'on obtient 9,3 g du composé attendu.

### C) 1-(2,4-Dichlorométhyl)-3-((1,3-dioxo-1,3-dihydro-2H-isoindol-2-yl)méthyl)-5-(4-méthoxy)-1H-pyrazole-4-carbonitrile.

A 9,3 g du composé obtenu à l'étape précédente dans 100 ml de DMF on ajoute 5,3 g de phtalimide de potassium et 3,5 g de NaI et on chauffe à 65°C pendant 2 heures et demie. Après retour à TA, on évapore le DMF puis le résidu est repris par AcOEt et on lave par une solution aqueuse de NaOH 1N. La phase organique est séchée sur Na₂SO₄, filtrée et évaporée à sec. Le résidu est repris par du DCM, lavé par une solution aqueuse de NaOH 1N puis par une solution saturée de NaCl. La phase organique est séchée sur Na₂SO₄, filtrée et évaporée à sec pour donner le composé attendu. (p = 10,53)

### D) Chlorhydrate de 3-(aminométhyl)-1-(2,4-dichlorophényl)-5-(4-méthoxyphényl)-1H-pyrazole-4-carbonitrile.

10,5 g du dérivé phtalimide obtenu à l'étape précédente est mis en suspension dans 250 ml d'éthanol, on ajoute 2,1 ml d'hydrazine monohydratée et on chauffe à reflux pendant 1 heure. On filtre le milieu réactionnel puis la phase organique est évaporée à sec. Le résidu est repris à l'éther et on ajoute une solution d'HCl dans l'éther chlorhydrique. Le précipité formé est filtré puis rincé au pentane. On obtient 5 g du composé attendu, F = 128°C.

### Préparation 2

1-(5-(4-Chlorophényl)-1-(2,4-dichlorophényl)-4-méthoxy-1*H*-pyrazol-3-yl méthanamine.

Les analyses LC/MS sont effectuées selon les conditions A.

### A) 1-(4-Chlorophényl)butane-1,3-dione.

On place sous azote un mélange contenant 45 g de 4-chlorobenzoate d'éthyle dans 235 ml de THF anhydre et 19,50 g de NaH (60 % dans l'huile minérale) dans 235 ml de THF anhydre. A 0°C, on ajoute goutte à goutte, 36 ml d'acétone et 750 ml de THF anhydre supplémentaire et on chauffe à reflux pendant 3 heures. On acidifie le milieu à pH = 5 par addition d'HCl 2N puis on extrait à l'éther et lave à l'eau puis par une solution saturée de NaHCO₃ ; on sèche sur MgSO₄ et concentre. Le produit brut est dissous dans un minimum de toluène, on filtre l'insoluble puis on purifie par chromatographie sur silice en éluant par un mélange cyclohexane/AcOEt (95/5 ; v/v). On obtient 47,9 g du composé attendu.
LC/MS : MH⁺ =197,0 ; tr = 9,67 mn.

### B) 2-Bromo-1-(4-chlorophényl)butane-1,3-dione.

On place sous azote 15,35 g du composé de l'étape précédente dans 20 ml de DCM et on ajoute, goutte à goutte à 0°C, 4,04 ml de brome. A la fin de l'addition, on évapore à sec puis on ajoute 300 ml de DCM ; on lave à l'eau, sèche sur Na₂SO₄ puis on filtre et évapore à sec. On obtient 21,15 g du composé attendu.

### C) Acétate de 1-(4-chlorobenzoyle)-2-oxopropyle.

On place sous azote 16,74 g d'acétate de potassium dissous à chaud dans 76,76 ml d'acide acétique. A 100°C, on ajoute, par portions, 21,5 g du dérivé bromé obtenu à l'étape précédente et on chauffe à 120°C pendant 3 heures. On verse le milieu réactionnel sur 1 litre d'eau et on extrait par 500 ml d'éther. La phase éthérée est lavée 2 fois par 250 ml d'une solution saturée de NaHCO₃ puis on sèche sur Na₂SO₄, filtre et évapore à sec. On obtient 16,28 g du composé attendu.
LC/MS : MH⁺ = 255,0 ; tr = 8,42 mn.

### D) Acétate de 5-(4-chlorophényl)-1-(2,4-dichlorophényl)-3-méthyl-1H-pyrazol-4-yl.

On chauffe à reflux pendant 3 heures un mélange contenant 16,23 g du composé de l'étape précédente et 13,92 g de chlorhydrate de (2,4-dichlorophényl)hydrazine. Après retour à TA, on ajoute 400 ml d'eau puis la phase organique est lavée par une solution saturée de NaHCO₃, puis à l'eau, puis séchée sur Na₂SO₄. On filtre et évapore à sec. Le produit obtenu est purifié par chromatographie sur silice en éluant par un mélange DCM/MeOH (98/2 ; v/v). On obtient 5,80 g du composé attendu (LC/MS : MH⁺ = 395,0 ; tr = 10,80 mn) et 4,63 g du composé désacétylé identique à celui préparé à l'étape suivante.

### E) 5-(4-Chlorophényl)-1-(2,4-dichlorophényl)-3-mémyl-1H-pyrazol-4-ol.

On mélange 16,2 g du composé obtenu à l'étape précédente dans 82 ml de méthanol avec 7,1 g de carbonate de potassium en solution dans l'eau (v/v) et on laisse sous agitation 4 heures à TA. Le milieu réactionnel est concentré puis on le dilue par addition de 500 ml d'eau et on extrait par 500 ml de DCM. La phase organique est lavée par une solution tampon à pH = 2 de l'eau distillée, puis séchée sur Na₂SO₄, filtrée et évaporée à sec. On obtient 11,56 g du composé attendu.
LC/MS:MH⁺ 355,0, tr = 9,69 mn.

### F) 5-(4-Chlorophényl)-1-(2,4-dichlorophényl)-4-méthoxy-3-méthyl-1H-pyrazole.

On place sous azote 16,25 g du composé obtenu à l'étape précédente en solution dans 200 ml de DMF, on ajoute 7,05 g de K₂CO₃ et 7,21 g de CH₃I puis on chauffe à 60°C sous azote, en agitant, pendant 3 heures. Après retour à TA, le milieu réactionnel est filtré. Le filtrat est additionné de 100 ml d'eau et extrait par 100 ml de DCM (2 fois). La phase organique est lavée par 100 ml d'eau (5 fois) puis séchée sur Na₂SO₄, filtrée et concentrée à sec. Le résidu est purifié sur une colonne de silice. On obtient 9,07 g du composé attendu.
LC/MS : MH⁺ = 367,0 ; tr = 11,10 mn.

### G) 3-Bromométhyl-5-(4-chlorophényl)-1-(2,4-dichlorophényl)-4-méthoxy-1H-pyrazole.

On place sous azote 9,07 g du composé obtenu à l'étape précédente dans 125 ml de CCl₄ et on ajoute 4,87 g de NBS, 0,79 g de péroxyde de benzoyle et 0,1 g de AIBN puis on chauffe à reflux pendant 60 heures. Après retour à TA, on filtre sur Célite^{®} et on évapore à sec puis on purifie par chromatographie sur silice en éluant par cyclohexane/AcOEt (95/5 ; v/v). A côté du composé monobromé (2,67 g) attendu, on obtient du 3,3-dibromo-5-(4-chlorophényl)-1-(2,4-dichlorophényl)-4-méthoxy-1*H-*pyrazole (5 g).
LC/MS : MH⁺ = 446,8 ; tr = 11,67 mn.
LC/MS : MH⁺ = 524,8 ; tr = 12,06 mn.

### H) 1-5-(4-Chlorophényl)-1-(2,4-dichlorophényl)-4-méthoxyl-1H-pyrazol-3-yl) méthanamine.

On place sous azote un mélange contenant 2,65 g du composé monobromé obtenu à l'étape précédente, 2,52 g d'hexaméthylène tétramine et 0,90 g de NaI dans 50 ml d'EtOH. On laisse sous agitation à TA pendant 18 heures, puis on ajoute 10 ml d'HCl concentré et 12 ml d'éthanol et on chauffe à reflux pendant 12 heures. Après retour à TA, le milieu est filtré puis le filtrat est évaporé à sec puis repris dans du DCM. La phase organique est extraite par 100 ml d'HCl à 10 %. La phase aqueuse est lavée au DCM puis basifiée et extraite au DCM. La phase organique est séchée sur Na₂SO₄, filtrée et évaporée à sec. Le résidu est purifié sur silice en éluant par DCM/MeOH (93/7 ; v/v). On obtient 1,47 g du composé attendu.
LC/MS : MH⁺ = 382,0 ; tr = 6,83 mn.

### I) 5-(4-Chlorophényl)-1-(2,4-dichlorophényl)-4-méthoxy-1H-pyrazole-3-carbaldéhyde.

Sous azote, on place dans 30 ml de DMSO 5 g du 3,3-dibromo-5-(4-chlorophényl)-1-(2,4-dichlorophényl)-4-méthoxy-1*H*-pyrazole obtenu à l'étape G, et on chauffe à 120°C pendant 6 heures. On verse le milieu réactionnel dans 100 ml d'eau et on extrait 2 fois par 100 ml d'AcOEt. On lave la phase organique par 100 ml de NaCl saturé et on sèche ensuite sur Na₂SO₄. Après évaporation à sec, on obtient 4 g du composé attendu non purifié.
LC/MS:MH⁺ = 381,0 ; tr = 11,06 mn.

### J) (5-(4-Clflorophényl)-1-(2,4-dichlorophényl)-4-inéthoxy-1H-pyrazol-3-yl) méthanol.

On place 4 g du composé obtenu à l'étape précédente dans 104 ml de méthanol et on ajoute à 0°C 0,99 g de NaBH₄ et on laisse sous agitation à 0°C pendant 45 minutes. On ajoute 3 ml d'AcOH pour décomposer NaBH₄ en excès. On évapore à sec puis on reprend le résidu par 100 ml de DCM, on lave par 100 ml de solution saturée de NaHCO₃ (2 fois) puis on sèche la phase organique et on la concentre à sec. On obtient 3,6 g du composé attendu non purifié.
LC/MS : MH⁺ = 3 83,0 ; tr = 9,68 mn.

### K) 2-((5-(4-Chlorophényl)-1-(2,4-dichlorophényl)-4-méthoxy-1H-pyrazol-3-yl) méthyl)-1H-isoindole-1,3-(2H)-dione.

On mélange 3,6 g du composé obtenu à l'étape précédente, 2,46 g de PPh₃ et 1,38 g de phtalimide en solution dans 156 ml de THF. A -10°C, on ajoute, goutte à goutte, 1,63 g de DEAD et on laisse une nuit à TA. On traite le milieu réactionnel par 100 ml d'une solution tampon à pH = 2 ; on dilue la phase organique par 200 ml d'éther puis on la lave par 140 ml de solution saturée de NaHCO₃ puis 100 ml de solution saturée de NaCl ; on sèche sur Na₂SO₄ ; on filtre et évapore à sec. Le produit obtenu est purifié sur silice en éluant par un mélange DCM/MeOH (98/2 ; v/v). On obtient 3,4 g du composé attendu.
LC/MS : MH⁺ = 512,0 ; tr = 11,43 mn.

### L) 1-(5-(4-Chlorophényl)-1-(2,4-dichlorophényl)-4-méthoxy-1H-pyrazol-3-yl) méthanamine.

On place sous azote 3,4 g du composé obtenu à l'étape précédente et 0,67 g d'hydrazine monohydratée en solution dans 95 ml de méthanol et on chauffe à reflux pendant 3 heures. Le milieu réactionnel est évaporé à sec, le résidu est repris par 150 ml d'éther ; la phase organique est lavée par une solution de NaOH à 10 % puis par une solution saturée de NaHCO₃ puis par une solution saturée de NaCl. On extrait au DCM puis on évapore à sec. On obtient 2,45 g du composé attendu, identique à celui obtenu à l'étape H.

### Préparation 3

1-(5-(4-Chlorophényl)-1-(2,4-dichlorophényl)-4-(tétrahydro-2*H*-pyran-2-yloxyméthyl)-1*H*-pyrazol-3-yl)méthanamine.

### A) 4-(Bromométhyl)-5-(4-chlorophényl)-1-(2,4-dichlorophényl)-1H-pyrazole-3-carboxylate de méthyle.

On place 19 g de 5-(4-chlorophényl)-1-(2,4-dichlorophényl)-4-méthyl-1*H-*pyrazole-3-carboxylate de méthyle dans 200 ml de CCl₄ et on ajoute 8,54 g de NBS puis 1 g de péroxyde de benzoyle et on chauffe à reflux pendant une nuit. Après retour à TA, on filtre le précipité formé et le lave par CCl₄. On évapore la totalité du filtrat puis on reprend par AcOEt et on lave par une solution saturée de NaCl (2 fois). On sèche sur MgSO₄ et évapore. Le composé attendu cristallise dans l'éther iso, on filtre et sèche pour obtenir 19,4 g du composé attendu.

### B) Acide 5-(4-chlorophényl)-1-(2,4-dichlorophényl)-4-hydroxyméthyl-1H-pyrazole-3-carboxylique.

On place 17 g du composé obtenu à l'étape précédente et 1,5 g de LiOH, H₂Odans 100 ml de THF et 50 ml d'eau et on chauffe 3 heures puis on laisse une nuit à TA sous agitation. On filtre le précipité formé puis on évapore le filtrat. Le résidu est repris par AcOEt puis on lave par une solution saturée de NaCl. On sèche sur MgSO₄, filtre et concentre le filtrat pour obtenir 11 g du composé attendu.

### C) 5-(4-Chlorophényl)-1-(2,4-dichlorophényl)-4-hydroxyméthyl-1H-pyrazole-3-carboxylate de méthyle.

On dissout 10 g de l'acide formé à l'étape précédente dans 100 ml de MeOH, on ajoute 1 ml de H₂SO₄ concentré et on chauffe 2 heures à reflux. Après refroidissement, on évapore le solvant puis on reprend par AcOEt. On lave par une solution de NaHCO₃ puis une solution saturée de NaCl et on sèche sur MgSO₄. On purifie par chromatographie en éluant par un mélange AcOEt/cyclohexane (10/90 puis 20/80 ; v/v). On obtient 2,8 g du composé attendu qui cristallise avec de l'éther iso.

### D) 5-(4-Chlorophényl)-1-(2,4-dichlorophényl)-4-(tétrahydro-2H-pyran-2-yloxyméthyl)-1H-pyrazole-3-carboxylate de méthyle.

On dissout 2,8 g du composé obtenu à l'étape précédente dans 48 ml de DCM, on ajoute 0,68 g de 3,4-dihydro-2*H*-pyrane et 0,07 g de PTSOH puis on laisse sous agitation à TA pendant 1 heure. On lave le milieu réactionnel par une solution de NaHCO₃ puis par une solution saturée de NaCl. On sèche sur MgSO₄ et évapore. Le produit obtenu est purifié par chromatographie sur silice en éluant par un mélange AcOEt/cyclohexane (5/95 puis 90/10 ; v/v). Le composé attendu cristallise dans le cyclohexane/AcOEt. On obtient 2,2 g.

### E) 2-(5-(4-Chlorophényl)-1-(2,4-dichlorophényl)-4-(tétrahydro-2H-pyran-2-yloxyméthyl)-1H-pyrazole-3-yl)méthyl)-1,3-bis(méthylène)isoindoline.

On dissout dans 50,5 ml de THF 2,6 g du composé obtenu à l'étape précédente, 0,97 g de phtalimide puis 1,74 g de PPh₃ et on ajoute, goutte à goutte à -10°C, 1,16 g de DEAD. On laisse revenir à TA puis on laisse sous agitation à TA pendant 96 heures. On extrait à l'éther et lave par une solution saturée de NaCl puis on sèche sur MgSO₄ et évapore. Le produit obtenu est purifié par chromatographie sur silice en éluant par AcOEt/cyclohexane (5/95 ; v/v). On obtient 2,2 g du composé attendu.

### F) 1-(5-(4-Chlorophényl)-1-(2,4-dichlorophényl)-4-(tétrahydro-2H-pyran-2-yloxyméthyl)-1H-pyrazol-3-yl)méthanamine.

On place 2,2 g du composé obtenu à l'étape précédente dans 40 ml de MeOH, on ajoute 0,40 ml d'hydrate d'hydrazine et on chauffe à reflux pendant 1 heure et demie. On laisse refroidir puis on évapore le solvant et on reprend par du DCM. On lave par une solution de NaOH à 10 % puis par une solution saturée de NaCl, on sèche sur MgSO₄ et évapore. On obtient 1,57 g du composé attendu sous forme brute.

### Préparation 4

1-(5-(4-Chlorophényl)-1-(2,4-dichorophényl)-4-(méthoxyméthyl)-1*H*-pyrazol-3-yl)méthanamine.

### A) 5-(4-Chlorophényl)-1-(2,4-dichlorophényl)-4-méthyl-1H-pyrazole-3-carboxylate de méthyle.

On place 20 g d'acide 1-(5-(4-chlorophényl)-1-(2,4-dichlorophényl)-4-méthyl)-1*H*-pyrazole-3-carboxylique dans 200 ml de MeOH, on ajoute 0,5 g de chlorure de toluène sulfonyle et on chauffe une nuit à reflux. On évapore à moitié puis on filtre le précipité formé. On lave à l'éther puis on sèche pour obtenir 20,6 g du composé attendu.

### B) 4-(Bromophényl)-5-(4-chlorophényl)-1-(2,4-dichlorophényl)-4-(bromométhyl)-1H-pyrazol-3-carboxylate de méthyle.

On place 16,5 g du composé obtenu à l'étape précédente dans 200 ml de CCl₄, on ajoute 7,42 g de NBS et 0,1 g de péroxyde de benzoyle puis on chauffe une nuit à reflux. On filtre le précipité formé puis on le lave par CCl₄. On évapore le solvant, reprend par DCM puis on lave la phase organique par de l'eau puis une solution saturée de NaCl. On sèche sur MgSO₄ et évapore. On fait cristalliser le composé attendu par DCM et éther iso. On obtient 1,3 g du composé attendu.

### C) 5-(4-Chlorophényl)-1-(2,4-dichlorophényl)-4-(méthoxyphényl)-1H-pyrazole-3-carboxylate de méthyle.

On lave 2,5 g de sodium par 100 ml du toluène puis 100 ml d'éther puis on le coupe en petits morceaux et on l'introduit dans 500 ml de MeOH. On ajoute à la solution de méthylate de sodium ainsi préparée 13 g du dérivé bromé obtenu à l'étape précédente et on agite pendant 30 minutes, on abandonne le milieu réactionnel à TA pendant 72 heures. Le précipité formé est filtré puis on évapore le filtrat. On traite par HCl à 25 %, on reprend par AcOEt. Après décantation, la phase organique est lavée par une solution saturée de NaCl (2 fois). On sèche sur Na₂SO₄ et concentre. Le produit attendu cristallise dans l'éther iso. On obtient 1 g du composé attendu.

### D) 5-(4-Chlorophényl)-1-(2,4-dichlorophényl)-4-méthoxyphényl-1H-pyrazole-3-yl)méthanol.

On dissout 5,8 g du composé obtenu à l'étape précédente dans 100 ml de THF et on additionne à 0°C par petites fractions 0,82 g de LiAlH₄. On laisse sous agitation à TA pendant 30 minutes puis on hydrolyse par addition de 15 ml de solution de NaOH 1N. On filtre le précipité formé puis on le lave avec du THF et évapore le filtrat. On reprend par AcOEt et lave par une solution saturée de NaCl. On sèche sur MgSO₄ et évapore. On fait cristalliser dans AcOEt/éther iso. On obtient 4,6 g du composé attendu.

### E) 3-(Chlorométhyl)-5-(4-chlorophényl)-1-(2,4-dichlorophényl)-4-méthoxyméthyl-1H-pyrazole.

On dissout 4,5 g du composé obtenu à l'étape précédente dans 50 ml de DCM puis on ajoute à 0°C 2,6 g de PCl₅ par petites fractions et on laisse sous agitation à TA pendant 1 heure. On ajoute 25 ml d'eau et on laisse une nuit à TA. Après décantation, la phase organique est lavée par une solution saturée de NaCl (2 fois) puis on sèche sur Na₂SO₄ et évapore. On effectue une chromatographie sur silice en éluant par un mélange AcOEt/cyclohexane (10/90 ; v/v). On obtient 2,17 g du composé attendu et 2 g du composé équivalent portant en position 3 le substituant dichlorométhyle.

### F) 1-(5-(4-Chlorophényl)-1-(2,4-dichlorophényl)-4-(méthoxyméthyl)-1H-pyrazol-3-yl)méthanamine.

On dissout 2 g du composé obtenu à l'étape précédente dans 50 ml de chloroforme et on ajoute 0,80 g d'hexaméthylènetétramine puis on laisse plusieurs jours sous agitation à TA. On évapore à moitié et on ajoute 50 ml d'éther. On filtre le précipité formé puis on le reprend par 100 ml d'EtOH et 15 ml de KCl concentré. On chauffe 2 heures à reflux puis on laisse une nuit à TA. On filtre le précipité de NH₄Cl formé. On évapore le filtrat, on le reprend par du DCM puis on lave par une solution de NaHCO₃ puis par une solution saturée de NaCl (2 fois). On sèche sur MgSO₄ et évapore. On reprend par de l'éther iso. On évapore à nouveau pour obtenir 1,7 g du composé attendu.
RMN : 7,1 : d : 2H ; 7,45 : d : 2H ; 7,55 : dd : 1 H ; 7,6 : d : 1 H ; 7,75 : d : 1 H.

### EXEMPLE 1 : Composé N° 52

3-Chloro-N-((5-(4-chlorophényl)-1-(2,4-dichlorophényl)-4-méthoxy-1*H*-pyrazol-3-yl)méthyl)benzènesulfonamide.

On mélange 0,32 g du composé obtenu à la Préparation 2 avec 0,09 g de triéthylamine et 0,19 g de 3-chlorobenzènesulfonyle dans 20 ml de dichlorométhane et on laisse une heure sous agitation à TA. On dilue par 100 ml de DCM puis on lave par une solution d'HCl à 10 % puis par une solution de NaOH à 25 %, puis par une solution saturée de NaCl et on extrait au DCM. On sèche, filtre et évapore à sec. Le résidu est purifié par chromatographie sur silice en éluant par un mélange DCM/MeOH (96/4 ; v/v). On obtient 0,21 g du composé attendu.

### EXEMPLE 2 : Composé N° 53

3-Chloro-N-((5-(4-chlorophényl)-1-(2,4-dichlorophényl)-4-hydroxyméthyl-1*H-*pyrazol-3-yl)méthyl)benzènesulfonamide.

### A) 3-Chloro-N-((5-(4-chlorophényl)-1-((tétrahydro-2H-pyran-2-yloxy)méthyl)-1H-pyrazol-3-yl)méthyl)benzènesulfonamide.

A partir de 0,7 g du composé obtenu à la Préparation 3, 0,35 g de 3-chlorobenzènesulfonyle et 0,33 g de triéthylamine, on obtient 0,847 g du composé attendu en suivant le mode opératoire décrit à l'Exemple précédent.

### B) 3-Chloro-N-((5-(4-chlorophényl)-4-(hydroxyméthyl)-1H-pyrazol-3-yl)méthyl)benzènesulfonamide.

On dissout 0,847 g du composé obtenu à l'étape précédente dans 40 ml de MeOH. On ajoute 1 ml d'HCl concentré et on chauffe à reflux pendant 10 minutes. On laisse refroidir puis on évapore le solvant et on reprend le résidu par AcOEt. On lave par une solution saturée de NaCl (2 fois) puis on sèche sur MgSO₄ et évapore. Le produit obtenu est purifié par chromatographie en éluant par un mélange AcOEt/cyclohexane (10/90 puis 20/80 puis 25/75 ; v/v). On obtient 176 mg du composé attendu et 289 mg du composé correspondant de formule (I) dans laquelle R₃ est un groupe méthoxyméthyle.

### EXEMPLE 3 : Composé 48

3-Chloro-N-((5-(4-Chlorophényl)-1-(2,4-dichlorophényl)-4-(méthoxyméthyl)-1*H*-pyrazol-3-yl)-méthyl)benzènesulfonamide.

On place 0,45 g d'amine obtenue à la Préparation 4 dans 20 ml de DCM. On ajoute 0,26 g de 3-chlorobenzènesulfonyle et 0,25 g de triéthylamine puis on laisse 3 heures sous agitation à TA. On ajoute 15 ml d'eau et on agite 10 minutes. Après décantation, la phase organique est lavée par une solution de NaHCO₃/KHSO₄ puis par une solution saturée de NaCl (2 fois). On sèche sur MgSO₄ et évapore. On reprend par de l'éther iso puis on purifie par une chromatographie sur silice en éluant par AcOEt/cyclohexane (5/95 ; v/v). On obtient 0,230 g du composé attendu, F = 154°C.

### EXEMPLE 4 : Composé 55

5-(4-Chlorophényl)-3-((((3-chlorophényl)sulfonyl)amino)méthyl)-1-(2,4-dichlorophényl)-N-méthyl-1*H*-pyrazole-4-carboxamide.

### A) Acide de 5-(4-chlorophényl)-3-((((3-chlorophényl)sulfonyl)amino)méthyl)-1-(2,4-dichlorophényl)-1H-pyrazole-4-carboxylique.

On ajoute 13,36 g de Cr₂O₃ sur un mélange de 11,5 ml de solution H₂SO₄ concentrée, diluée dans 50 ml d'eau froide pour préparer le réactif de Jones. On dissout 0,5 g du composé de l'Exemple 2 dans 15 ml d'acétone puis on ajoute lentement à une température comprise entre 0°C et 5°C, 5 ml du réactif de Jones. On laisse 2 jours sous agitation. On ajoute 10 ml d'isopropanol pour détruire l'excès de réactif et on filtre le précipité formé. On extrait le filtrat par 30 ml d'éther. La phase organique est lavée par une solution saturée de NaCl (2 fois) puis séchée sur MgSO₄. On évapore et reprend par de l'éther iso à chaud. Le compsoé attendu cristallise. On obtient 440 mg, F = 211 °C.

### B) 5-(4-Chlorophényl)-3-((((3-chlorophényl)sulfonyl)amino)méthyl)-1-(2,4-dichlorophényl)-N-méthyl-1H-pyrazole-4-carboxamide.

On dissout 0,4 g d'acide obtenu à l'étape précédente dans 20 ml de DCM, on ajoute 0,37 g de BOP et 0,05 g de méthylamine et on laisse sous agitation une nuit à TA. On ajoute 10 ml d'eau puis la phase organique est décantée. On évapore puis on reprend le résidu par AcOEt. On lave successivement par des solutions de K₂SO₄, NaHCO₃ et NaCl (solution saturée), on sèche sur MgSO₄ et évapore à sec. Le résidu est repris à chaud par Et₂O puis on cristallise. On obtient 0,35 g du composé attendu, F = 178°C.

### EXEMPLE 5 : Composé N°57

On place 0,69 g du composé de l'Exemple 1 dans 15 ml de DCM, à -20°C on ajoute 12,36 mg de BBr₃ puis on laisse sous agitation 1 heure à -20°C puis 3 heures à TA. On ajoute au milieu réactionnel 100 ml d'eau et 100 ml de DCM, on décante puis on lave la phase organique par 100 ml d'HCl dilué ; on sèche sur MgSO₄ et évapore à sec. Le résidu est repris par 100 ml de DCM. Le composé attendu précipite, on obtient 373 mg de produit attendu.
LC/MS : MH = 541,8 ; tr = 10,69 mn.

### EXEMPLE 6:

Les composés de formule (I_{A})décrits dans le tableau 1 sont préparés soit selon les procédés décrits ci-dessus, soit par chimie combinatoire selon le procédé décrit ci-après.

On dissout le dérivé du pyrazolméthylamine de formule (II) dans le DMF à la concentration de 0,1M en présence de 3 équivalents de DIPEA. Dans chaque puits de 2 ml, on place 300 µl de cette solution et on ajoute 120 µl de solution de chlorure de sulfonyle (R₁SO₂Cl) à la concentration de 0,25M dans le THF. Les plaques sont agitées à TA pendant 16 heures puis évaporées. Les produits formés sont dissous dans chaque puits par 500 µl d'AcOEt, on ajoute 400 µl de Na₂CO₃ 0,1M et les plaques sont agitées. Après décantation, 350 µl de phase aqueuse sont écartés puis on ajoute 40 µl de DMF puis 300 µl de CH₃CN.

### EXEMPLE 7 : Composé N° 45

N-((4-cyano-1-(2,4-dichlorophényl)-5-(4-méthoxyphényl)-1*H*-pyrazol-3-yl)méthyl)-2,6-difluorobenzenesulfonamide.

A une solution contenant 1,2 g du composé de la Préparation 1, on ajoute 0,98 ml de triéthylamine et 0,75 g de chlorure de 2,6-difluorobenzènesulfonyle en solution dans le DCM. Après une nuit sous agitation à TA, on concentre le DCM, reprend le résidu par AcOEt, lave la phase organique par une solution tampon pH2 puis par une solution saturée de NaHCO₃ puis par une solution saturée de NaCl ; on sèche sur MgSO₄ et concentre à sec sous vide. On purifie par chromatographie sur silice en éluant par un mélange cyclohexane/AcOEt (90/10 ; v/v). On obtient 1,5 g du composé attendu. F =170°C.

Les tableaux qui suivent illustrent les structures chimiques et les propriétés physiques de quelques composés selon l'invention.

Dans ces tableaux, Me, tBu illustrent respectivement les groupes méthyle et *tert-*butyle.

**TABLEAU 1**

| | | | | |
|---|---|---|---|---|
| 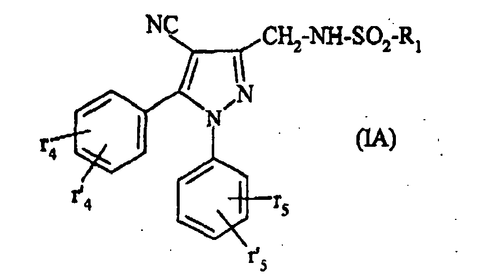 | | | | |

| Composés | r₄, r'₄ | r₅, r'₅ | R₁ | Caractérisation Conditions F°C |
|---|---|---|---|---|
| 1 | 4-Cl | 2,4-diCl | 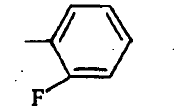 | MH⁺ = 534,8 tr = 2,00 MS5 |
| 2 | 4-Cl | 2,4-diCl | 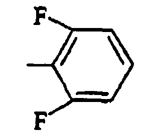 | MH⁺ = 552,7 tr = 2,04 MS5 |
| 3 | 4-Cl | 2,4-diCl | 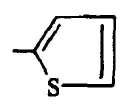 | MH⁺ = 522,7 tr = 2,01 MS5 F = 184 |
| 4 | 4-Cl | 2,4-diCl | 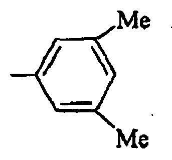 | MH⁺ = 544,8 tr = 2,13 MS5 |
| 5 | 4-Cl | 2,4-diCl | 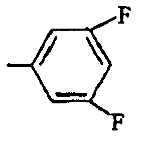 | MH⁺ = 552,7 tr = 2,08 MS5 |
| 6 | 4-Cl | 2,4-diCl | 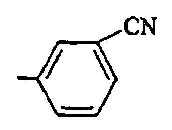 | MH⁺ = 541,8 tr = 2,03 MS5 F = 167 |
| 7 | 4-Cl | 2,4-diCl | 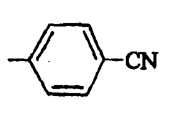 | MH⁺ = 541,8 tr = 1,99 MS5 F = 187 |
| 8 | 4-Cl | 2,4-diCl | 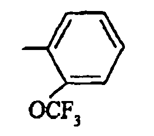 | MH⁺ = 600,7 tr = 2,12 MS5 F = 126 |
| 9 | 4-Cl | 2,4-diCl | 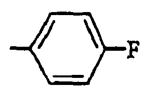 | MH⁺ = 534,8 tr = 2,03 MS5 |
| 10 | 4-Cl | 2,4-diCl | 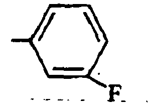 | MH⁺ = 534,8 tr = 2,07 MS5 |
| 11 | 4-Cl | 2,4-diCl | 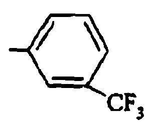 | MH = 584,7 tr = 2,09 MS5 F = 104 |
| 12 | 4-Cl | 2,4-diCl | 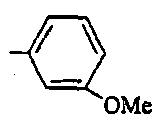 | MH⁺ = 546,8 tr = 2,07 MS5 F =141 |
| 13 | 4-Cl | 2,4-diCl | 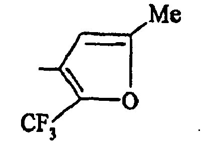 | MH⁺ = 588,7 tr = 2,14 MS5 F= 184 |
| 14 | 4-Cl | 2,4-diCl | 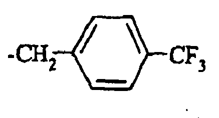 | MH⁺ = 598,8 tr = 2,11 MS5 F = 152 |
| 15 | 4-Cl | 2,4-diCl | 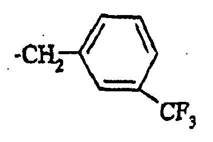 | MH⁺ = 598,8 tr = 2,13 MS5 F = 89 |
| 16 | 4-Cl | 2,4-diCl | 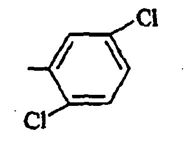 | MH⁺ = 584,7 tr = 2,13 MS5 F = 165 |
| 17 | 4-Cl | 2,4-diCl | 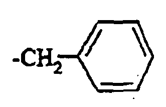 | MH⁺ = 530,8 tr = 2,08 MS5 F = 93 |
| 18 | 4-Cl | 2,4-diCl | 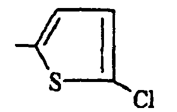 | MH⁺ = 556,7 tr = 2,13 MS5 |
| 19 | 4-Cl | 2,4-diCl | 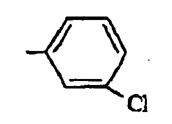 | MH⁺ = 550,7 tr = 2,11 MS5 F = 98 |
| 20 | 4-Cl | 2,4-diCl | 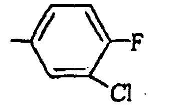 | MH⁺ = 568,7 tr = 2,10 MS5 |
| 21 | 4-Cl | 2,4-diCl | 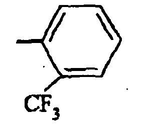 | MH⁺ = 584,7 tr = 2,10 MS5 |
| 22 | 4-Cl | 2-Cl | 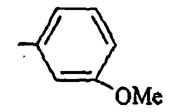 | MH⁺ = 513,5 tr = 1,82 MS5 |
| 23 | 4-Cl | 2-Cl | 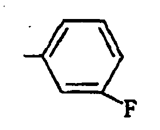 | MH⁺ = 501,5 tr = 1,83 MS2 F=85 |
| 24 | 4-Cl | 2-Cl | 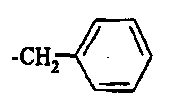 | MH⁺ = 497,5 tr = 1,83 MS2 |
| 25 | 4-Cl | 2-Cl | 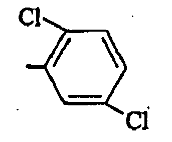 | MH⁺ = 551,4 tr = 1,90 MS2 F = 110 |
| 26 | 4-Cl | 2-Cl | 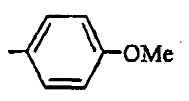 | MH⁺ = 513,5 tr = 1,80 MS2 |
| 27 | 4-Cl | 2-Cl | 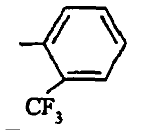 | MH⁺ = 551,5 tr = 1,86 MS2 |
| 28 | 4-Cl | 2-Cl | 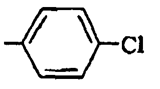 | MH⁺ = 517,4 tr = 1,87 MS2 |
| 29 | 4-Cl | 2-Cl | 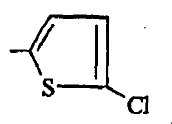 | MH⁺ = 523,4 tr = 1,87 MS2 F = 118 |
| 30 | 4-Cl | 2-Cl | 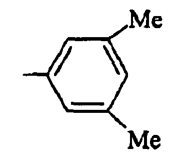 | MH⁺ = 511,5 tr = 1,89 MS2 F = 154 |
| 31 | 4-Cl | 2-Cl | 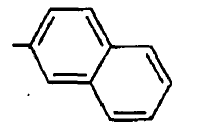 | MH⁺ = 533,5 tr = 1,88 MS2 |
| 32 | 4-Cl | 2-Cl | 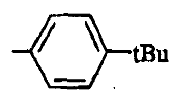 | MH⁺ = 539,5 tr = 1,96 MS2 |
| 33 | 4-Cl | 2-Cl | 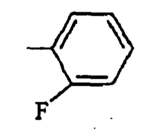 | MH⁺ = 501,5 tr = 1,81 MS2 F=75 |
| 34 | 4-Cl | 2-Cl | 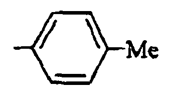 | MH⁺ = 497,5 tr = 1,84 MS2 |
| 36 | | | 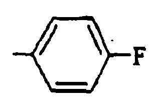 | MH⁺ = 501,5 tr = 1,82 MS2 |
| | 4-Cl | 2-Cl | 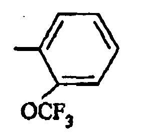 | MH⁺ = 567,5 tr = 1,88 MS2 F = 87 |
| 37 | 4-Cl | 2-Cl | 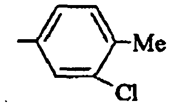 | MH = 531,5 tr = 1,90 MS2 |
| 38 | 4-Cl | 2-Cl | 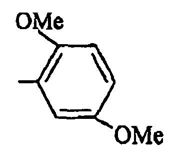 | MH⁺ = 543,5 tr = 1,81 MS2 |
| 39 | 4-Cl | 2-Cl | 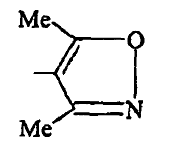 | MH⁺ = 502,5 tr = 1,80 MS2 |
| 40 | 4-Cl | 2-Cl | 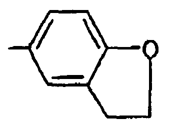 | MH⁺ = 525,5 tr = 1,81 MS2 |
| 41 | 4-Cl | 2-Cl | 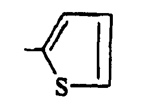 | MH⁺ = 489,4 tr = 1,80 MS2 |
| 42 | 4-Cl | 2-Cl | 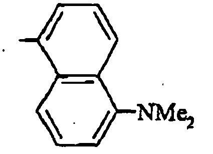 | MH⁺ = 576,5 tr = 1,84 MS2 |
| 43 | 4-Cl | 2-Cl | 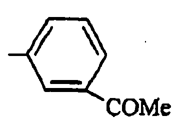 | MH⁺ = 525,5 tr = 1,78 MS5 F=117 |
| 44 | 4-OMe | 2,4-diCl | 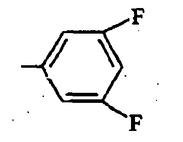 | MH⁺ = 548,8 tr = 6,67 MS5 F = 153 |
| 45 | 4-OMe | 2,4-diCl | 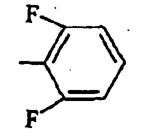 | MH⁺ = 548,8 tr = 6,39 MS5 F=170 |
| 46 | 4-OMe | 2,4-diCl | 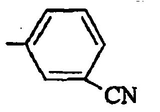 | MH⁺ = 537,8 tr = 6,35 MS5 |
| 47 | 4-OMe | 2,4-diCl | 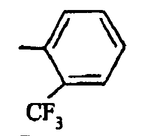 | MH⁺ = 580,8 tr = 6,80 MS5 |

**TABLEAU 2**

| | | | |
|---|---|---|---|
| 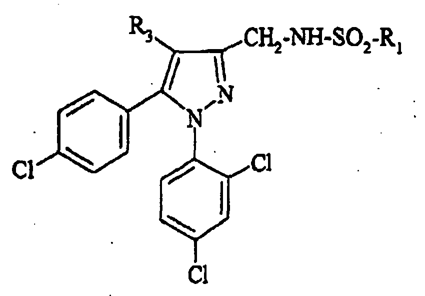 | | | |

| Composés | R₁ | R₃ | Caractérisation Conditions F°C |
|---|---|---|---|
| 48 | 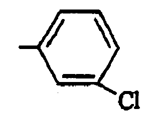 | -CH₂OMe | F = 154°C |
| 49 | 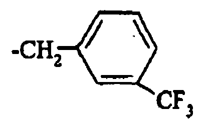 | -CH₂OMe | F = 74°C |
| 50 | 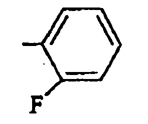 | -CH₂OMe | F = 143°C |
| 51 | 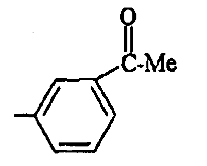 | -CH₂OMe | F = 85°C |
| 52 | 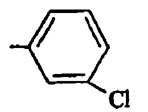 | -OMe | MH⁺ = 556,0 tr = 11,48 |
| 53 | 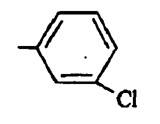 | -CH₂OH | F = 83°C |
| 54 | 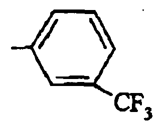 | -CH₂OH | F = 82°C |
| 55 | 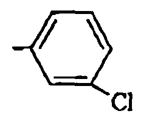 | 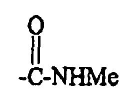 | F = 178°C |
| 56 | 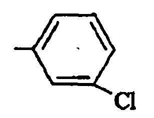 | 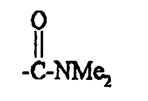 | F = 164°C |
| 57 | 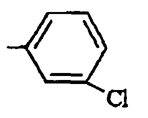 | -OH | MH⁺ = 541,8 tr = 10,69 |
| 58 | 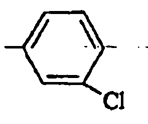 | 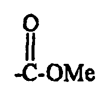 | F = 132°C |
| 59 | 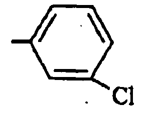 | -CH₂OEt | F =143°C |
| 60 | 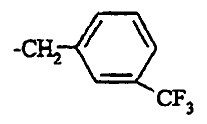 | 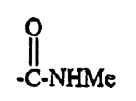 | F = 98°C |
| 61 | 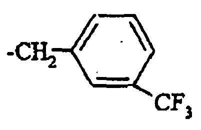 | 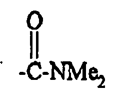 | F = 83°C |
| 62 | 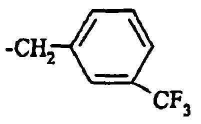 | 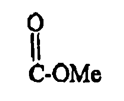 | F = 125°C |
| 63 | 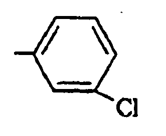 | -CH₂CN | |
| 64 | 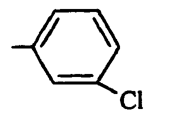 | 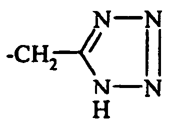 | |
| 65 | 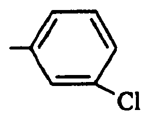 | 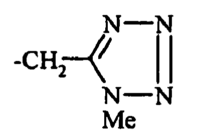 | |
| 66 | 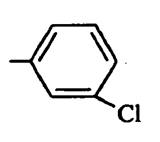 | 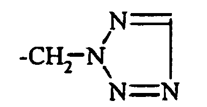 et 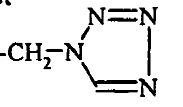 | Mélange d'isomères MH⁺ = 607 tr₁ = 10,52 tr₂ = 10,99 |
| 67 | 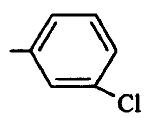 | 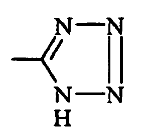 | |
| 68 | 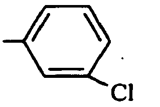 | 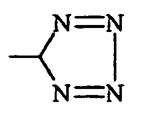 | |

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997,272, 22330-22339.

L'interaction d'un composé selon l'invention avec les récepteurs CB1 présents dans le cerveau est déterminée chez la souris avec le test de binding ex vivo du [3H]-CP55940 après une injection intraveineuse comme décrit dans M. Rinaldi-Cannona et al., FEBS Letters, 1994, 350, 240-244 et M. Rinaldi-Carmona et al., Life Sciences, 1995, 56, 1941-1947.

L'interaction d'un composé selon l'invention avec les récepteurs CB1 présents à la périphérie est déterminée chez la souris avec le test de réversion de l'effet inhibiteur du CP55940 sur transit gastrointestinal après une administration orale comme décrit dans M. Rinaldi-Carmona et al., J. Pharmacol. Exp. Ther., 2004, 310, 905-914.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable, ou encore un solvat ou un hydrate du composé de formule (I).

Ainsi les composés selon l'invention peuvent être utilisés dans le traitement ou la prévention de maladies impliquant les récepteurs aux cannabinoïdes CB₁ chez l'homme ou chez l'animal notamment chez les mammifères incluant de façon non limitative les chiens, les chats, les chevaux, les bovins, les moutons.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles du déficit de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans la prévention et le traitement des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie telles que la cirrhose chronique, la fibrose, la stéatose hépatique, la stéatohépatite; ainsi que des troubles d'origine endocrinienne, des troubles cardio-vasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles de la fertilité, de l'accouchement prématuré, de l'interruption de grossesse, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose.

Selon la présente invention, les composés de formule (I) sont tout particulièrement utiles pour la préparation de médicaments utiles pour la prévention et le traitement des troubles psychotiques, en particulier la schizophrénie, les troubles du déficit de l'attention et de l'hyperactivité (TDAH) chez les enfants hyperkinétiques; pour la prévention et le traitement des déficits mnésiques et cognitifs ; de la dépendance alcoolique, de la dépendance nicotinique, du sevrage alcoolique et du sevrage tabagique ; des maladies neurodégénératives aiguës ou chroniques.

Plus particulièrement, les composés de formule (I) selon la présente invention sont utiles dans le traitement et la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), de ses sels pharmaceutiquement acceptables et de leurs solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :
- un autre antagoniste des récepteurs CB₁ aux cannabinoïdes ;
- un modulateur des récepteurs CB₂ aux cannabinoïdes ;
- un antagoniste des récepteurs AT₁ de l'angiotensine II ;
- un inhibiteur de l'enzyme de conversion ;
- un antagoniste calcique;
- un diurétique ;
- un béta-bloquant ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antispychotique, un anxiolytique ;
- un anticancéreux ou un agent antiprolifératif ;
- un antagoniste des opioïdes ;
ainsi que :
- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs AT₁ de l'angiotensine II, on entend un composé tel que candésartan cilexitil, éprosartan, irbésartan, losartan potassium, olmésartan médoxomil, telmisartan, valsartan, chacun de ces composés pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril, lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, nifénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibornuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité ou agissant sur les troubles du métabolisme, on entend un composé tel que amfépramone, benfluorex, benzphétamine, indanorex, mazindole, méfénorex, méthamphétamine, D-norpseudoéphédrine, sibutramine, un topiramate, un inhibiteur de lipase (orlistat cetilistat), un agoniste PPAR (de l'anglais Peroxisome Proliferator Activated Receptor Agonist), un agoniste de la dopamine, un agoniste des récepteurs de leptine, un inhibiteur du reuptake de la sérotonine, un béta-3 agoniste, un CCK-A agoniste, un inhibiteur de NPY,un agoniste des récepteurs MC4, un antagoniste des récepteurs MCH (de l'anglais Melanin Concentrating Hormone), un antagoniste de l'orexine, un inhibiteur de phosphodiesterase, un inhibiteur de 11βHSD (11-β-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un modulateur des transporteurs de phosphate, de glucose, d'acide gras, de dicarboxylate, un antagoniste 5HT₂, un antagoniste 5HT₆, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon la présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :
inhibiteurs de PTP 1 B (de l'anglais Protein Tyrosine Phosphase -1B), les agonistes des récepteurs VPAC 2, les modulateurs de GLK, les modulateurs retinoides, les inhibiteurs de glycogen phosporylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalene synthetase, les inhibiteurs de squalene epoxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript),les modulateurs MC 4 (mélanocortin 4), les antagonistes des récepteurs de l'oréxine.

Selon un autre aspect de l'invention, le composé de formule (I), un de ses sels pharmaceutiquement acceptables ou un de leurs solvats et l'autre principe actif associé peuvent être administrés de manière simultanée, séparée ou étalée dans le temps.

On entend par «utilisation simultanée » l'administration des composés de la composition selon l'invention compris dans une seule et même forme pharmaceutique.

On entend par «utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratracheale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 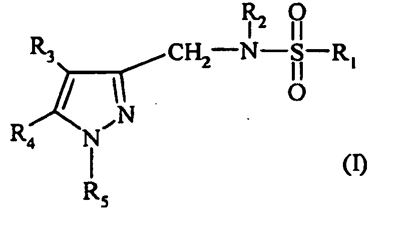 dans laquelle :
- R₁ représente
. un phényle non substitué ou substitué par un ou plusieurs substituants choisis indépendamment parmi un atome d'halogène, un (C₁-C₄)alkyle, un (C₁-C₄)alcoxy, un cyano, un groupe trifluorométhyle, un groupe trifluorométhoxy, un groupe S(O)ₙAlk, un groupe (C₁-C₄)alkylcarbonyle, un phényle ;
. un benzyle non substitué ou substitué par un ou plusieurs substituants choisis indépendamment parmi un atome d'halogène, un (C₁-C₄)alkyle, un (C₁-C₄)alcoxy ; un groupe trifluorométhyle ;
. un radical thiényle, furyle, oxazolyle, thiazolyle, imidazolyle, ledit radical étant non substitué ou substitué par un ou plusieurs substituants choisis indépendamment parmi un atome d'halogène, un (C₁-C₄)alkyle, un groupe trifluorométhyle ;
. un naphtyle non substitué ou substitué par un ou plusieurs substituants choisis indépendamment parmi un (C₁-C₄)alkyle, un di(C₁-C₄)alkylamino ;
. un 2,3-dihydrobenzofuran-yle non substitué ou substitué une ou plusieurs fois par un groupe (C₁-C₄)alkyle ;
- R₂ représente un atome d'hydrogène ou un (C₁-C₄)alkyle ;
- R₃ représente un cyano, un hydroxyle, un (C₁-C₄)alcoxy, un cyanométhyle, un hydroxyméthyle, un (C₁-C₄)alcoxyméthyle, un fluorométhyle, un tétrazolylméthyle, un N-(méthyl)tétrazolylméthyle, un tétrazolyle, un N-(méthyl)tétrazolyle, un groupe CONR₆R₇, un groupe CH₂S(O)ₙAlk, un groupe COOR₈ ;
- R₄ et R₅ représentent chacun indépendamment un phényle non substitué ou substitué par un ou plusieurs substituants choisis indépendamment parmi un atome d'halogène, un (C₁-C₇)alkyle, un (C₁-C₄)alcoxy, un groupe trifluorométhyle ou un groupe S(O)ₙAlk ;
- R₆ et R₇ représentent chacun indépendamment un atome d'hydrogène ou un (C₁-C₄)alkyle ou R₆ et R₇ ensemble avec l'atome d'azote auquel ils sont liés constituent un radical hétérocyclique choisi parmi pyrrolidinyle, pipéridinyle, morpholinyle, pipérazinyle non substitué ou substitué une ou plusieurs fois par un (C₁-C₄)alkyle ;
- R₈ représente un (C₁-C₄)alkyle ;
- n représente 0, 1 ou 2 ;
- Alk représente un (C₁-C₄)alkyle ;
à l'état de base ou de sels d'addition à des acides, ainsi qu'à l'état d'hydrates ou de solvats.

2. Composé selon la revendication 1 de formule I_{A} dans laquelle R₃ est un cyano et les substituants R₁, R₂, R₄, R₅ sont tels que définis pour les composés de formule (I) à la revendication 1.

3. Composé selon la revendication 1 de formule I_{B} dans laquelle R₃ est un hydroxyle et les substituants R₁, R₂, R₄, R₅ sont tels que définis pour les composés de formule (I) à la revendication 1.

4. Composé selon la revendication 1 de formule I_{C} dans laquelle R₃ est un (C₁-C₄)alcoxy et les substituants R₁, R₂, R₄, R₅ sont tels que définis pour les composés de formule (I) à la revendication 1.

5. Composé selon la revendication 1 de formule I_{F} dans laquelle R₃ est un (C₁-C₄)alcoxyméthyle et les substituants R₁, R₂, R₄, R₅ sont tels que définis pour les composés de formule (I) à la revendication 1.

6. Composé selon la revendication 1 de formule I_{I} dans laquelle R₃ est un groupe CONR₆R₇ et les substituants R₁, R₂, R₄, R₅, R₆ et R₇ sont tels que définis pour les composés de formule (I) à la revendication 1.

7. Composé selon la revendication 1 de formule I_{J} dans laquelle R₃ est un COOR₈ et les substituants R₁, R₂, R₄, R₅ et R₈ sont tels que définis pour les composés de formule (I) à la revendication 1.

8. Composé selon la revendication 1 de formule I_{O} dans laquelle R₃ est un tétrazol-1-ylméthyle ou un tétrazol-2-ylméthyle et les substituants R₁, R₂, R₄, R₅ sont tels que définis pour les composés de formule (I) à la revendication 1.

9. Composé selon la revendication 1 de formule I dans laquelle :
- R₁ représente :
. un radical phényle, dans lequel chaque groupe phényle est non substitué ou substitué une ou plusieurs fois par des substituants choisis indépendamment parmi un atome d'halogène, un (C₁-C₄)alkyle, un (C₁-C₄)alcoxy, un trifluorométhyle ;
. un radical furyle non substitué ou substitué par un ou plusieurs substituants choisis indépendamment parmi un atome d'halogène, un (C₁-C₄)alkyle, un groupe trifluorométhyle ;
- R₂ représente un atome d'hydrogène ;
- R₃ représente un cyano, un hydroxyle, un (C₁-C₄)alcoxy, un hydroxyméthyle, un (C₁-C₄)alcoxyméthyle, un groupe CON-R₆R₇, un groupe COOR₈, un tétrazol-1-yl méthyle ou un tétrazol-2-ylméthyle, les groupes R₆, R₇, R₈ étant tels que définis pour (I) ;
- R₄ représente un 4-chlorophényle, un 4-méthoxyphényle ;
- R₅ représente un 2-chlorophényle, un 2-bromophényle ou un 2,4-dichlorophényle ;
à l'état de base ou de sel d'addition à des acides ainsi qu'à l'état d'hydrates ou de solvats.

10. Composé selon la revendication 1 de formule I dans laquelle :
- R₁ représente un groupe 3-chlorophényle, 3-fluorophényle, 3,6-difluorophényle, 2,6-difluorophényle, 3-méthoxyphényle, 3-trifluorométhylphényle, 3-trifluorométhoxyphényle, un benzyle, un 4-trifluorométhylbenzyle ou un 2-trifluorométhyl-4-méthylfuryle ;
- R₂ représente un atome d'hydrogène ;
- R₃ représente un groupe cyano, méthoxy ou diméthylaminocarbonyle ;
- R₄ représente un 4-chlorophényle, un 4-méthoxyphényle ;
- R₅ représente un 2,4-dichlorophényle ou un 2-chlorophényle ;
à l'état de base ou de sel d'addition à des acides ainsi qu'à l'état d'hydrates ou de solvats.

11. Composé selon la revendication 1, choisi parmi :
- le N-{[5-(-4-chlorophényl)-4-cyano-1-(2,4-dichlorophényl)-1*H*-pyrazol-3 yl]méthyl}-3-cyanobenzènesulfonamide,
- le N-{[5-(4-chlorophényl)-4-cyano-1-(2,4-dichlorophényl)-1*H*-pyrazol-3-yl]méthyl}-3-trifluorobenzènesulfonamide,
- le N-{[5-(4-chlorophényl)-4-cyano-1-(2,4-dichlorophényl)-1*H*-pyrazol-3-yl]méthyl}-2-trifluorométhoxybenzènesuifonamide,
- le N-{[5-(4-chlorophényl)-4-cyano-1-(2,4-dichlorophényl)-1*H*-pyrazol-3-yl]méthyl}-3-méthoxybenzènesulfonamide,
- le N-{[5-(4-chlorophényl)-4-cyano-1-(2,4-dichlorophényl)-1*H*-pyrazol-3-yl]méthyl} -3-chlorobenzènesulfonamide,
- le N-{[5-(4-chlorophényl)-4-cyano-1-(2-chlorophényl)-1*H*-pyrazol-3-yl]méthyl}-3-fluorobenzènesulfonamide,
- le N-{[5-(4-chlorophényl)-4-cyano-1-(2-chlorophényl)-1*H*-pyrazol-3-yl]méthyl}-2-fluorobenzènesulfonamide,
- le N-{[5-(4-chlorophényl)-4-cyano-1-(2-chlorophényl)-1*H*-pyrazol-3-yl]méthyl}-2-trifluorométhoxybenzènesulfonamide,
- le N-{[5-(4-méthoxyphényl)-4-cyano-1-(2,4-dichlorophényl)-1*H*-pyrazol-3-yl]méthyl}-3,5-difluorobenzènesulfonamide,
- le N-{[5-(4-chlorophényl)-4-méthoxy-1-(2,4-dichlorophényl)-1*H*-pyrazol-3-yl]méthyl}-3-chlorobenzènesulfonamide,
- le 5-(4-chlorophényl)-3-({[(3-chlorophényl)sulfonyl]amino}méthyl)-1-(2,4-dichlorophényl)-*N*-méthyl-1*H*-pyrazole-4-carboxamide,
- le N-{[5-(4-chlorophényl)-4-cyano-1-(2,4-dichlorophényl)-1*H*-pyrazol-3-yl]méthyl}-1-phénylméthanesulfonamide,
- le N-{[5-(4-chlorophényl)-4-cyano-1-(2,4-dichlorophényl)-1*H*-pyrazol-3-yl]méthyl}-5-méthyl-2-(trifluorométhyl)furane-3-sulfonamide ;
à l'état de base ou de sel d'addition à des acides ainsi qu'à l'état d'hydrates ou de solvats.

12. Procédé de préparation d'un composé de formule (I) selon la revendication 1 **caractérisé en ce que** l'on fait réagir, en présence d'une base, dans un solvant, un composé de formule : 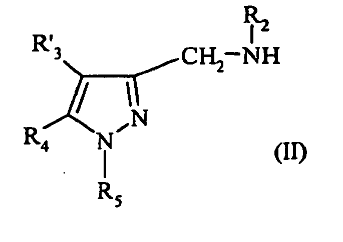 dans laquelle R₂, R₄, R₅ sont tels que définis pour un composé de formule (I) et R'₃ représente R₃ ou un précurseur de R₃, avec un halogénure de sulfonyle de formule HalSO₂R₁, dans laquelle R₁ est tel que défini pour un composé de formule (I) à la revendication 1 et Hal représente un atome d'halogène ; et le cas échéant, on transforme le composé obtenu de formule : 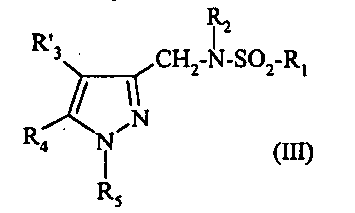 dans laquelle R'₃ est R₃ ou est un précurseur de R₃, en un composé de formule (I).

13. Médicament **caractérisé en ce qu'**il comprend un composé de formule (I) selon l'un quelconque des revendications 1 à 11, ou un hydrate ou un solvat d'un composé de formule (I).

14. Composition pharmaceutique, **caractérisée en ce qu'**elle comprend un composé de formule (I) selon l'une quelconque des revendications 1 à 11, ou un hydrate ou un solvat de ce composé, ainsi qu'au moins un excipient pharmaceutiquement acceptable.

15. Composé de formule (I) tel que défini dans les revendications 1 à 11, pour le traitement ou la prévention des maladies dans lesquelles les récepteurs CB₁ sont impliqués.

16. Composé selon la revendication 15 **caractérisé en ce que** les maladies sont les désordres psychiatriques, la dépendance et le sevrage à une substance, les troubles cognitifs, les troubles de l'attention et de la vigilance, les maladies neurodégénératives aiguës et chroniques.

17. Composé selon la revendication 15 **caractérisé en ce que** les maladies sont les troubles du métabolisme, les troubles de l'appétance, les troubles de l'appétit, l'obésité, le diabète de type II, le syndrome métabolique, la dyslipidémie.

18. Composé selon la revendication 15 **caractérisé en ce que** les maladies sont la douleur, la douleur neuropathique, la douleur induite par le traitement anticancéreux.

19. Composé selon la revendication 15 **caractérisé en ce que** les maladies sont les troubles gastro-intestinaux, les vomissements, les troubles diarrhéiques, l'ulcère, les maladies du foie.

20. Composé selon la revendication 15 **caractérisé en ce que** les maladies sont les maladies du système immunitaire, arthrite rhumatoïde, la démyélinisation, la sclérose en plaques, les maladies inflammatoires.

21. Composé selon la revendication 15 **caractérisé en ce que** les maladies sont les maladies d'Alzheimer, de Parkinson, la schizophrénie, les troubles cognitifs, le diabète, l'obésité, le syndrome métabolique et le sevrage tabagique.

22. Utilisation d'un composé selon l'une quelconque des revendications 1 à 11 pour la préparation d'un médicament pour le traitement ou la prévention des désordres psychiatriques, de la dépendance et du sevrage à une substance, des troubles cognitifs, des troubles de l'attention et de la vigilance, des maladies neurodégénératives aiguës et chroniques, des troubles du métabolisme, des troubles de l'appétance, des troubles de l'appétit, de l'obésité, du diabète de type II, du syndrome métabolique, de la dyslipidémie, de la douleur, de la douleur neuropathique, de la douleur induite par le traitement anticancéreux, des troubles gastro-intestinaux, des vomissements, des troubles diarrhéiques, de l'ulcère, des maladies du foie, des maladies du système immunitaire, de l'arthrite rhumatoïde, de la démyélinisation, de la sclérose en plaques, des maladies inflammatoires, des maladies d'Alzheimer, de Parkinson, de la schizophrénie, des troubles cognitifs, du diabète, de l'obésité, du syndrome métabolique et du sevrage tabagique.

## Claims

1. Compound corresponding to the formula (I): 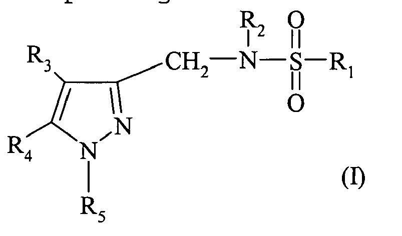 in which:
- R₁ represents
. a phenyl unsubstituted or substituted with one or more substituents selected independently from a halogen atom, a (C₁-C₄)alkyl, a (C₁-C₄)alkoxy, a cyano, a trifluoromethyl group, a trifluoromethoxy group, an S(O)ₙAlk group, a (C₁-C₄)alkylcarbonyl group, a phenyl;
. a benzyl unsubstituted or substituted with one or more substituents selected independently from a halogen atom, a (C₁-C₄)alkyl, a (C₁-C₄)alkoxy; a trifluoromethyl group;
. a thienyl, furyl, oxazolyl, thiazolyl, imidazolyl radical, said radical being unsubstituted or substituted with one or more substituents selected independently from a halogen atom, a (C₁-C₄)alkyl, a trifluoromethyl group;
. a naphthyl unsubstituted or substituted with one or more substituents selected independently from a (C₁-C₄)alkyl, a di(C₁-C₄)alkylamino;
. a 2,3-dihydrobenzofuranyl unsubstituted or substituted one or more times with a (C₁-C₄)alkyl group;
- R₂ represents a hydrogen atom or a (C₁-C₄)alkyl;
- R₃ represents a cyano, a hydroxyl, a (C₁-C₄)alkoxy, a cyanomethyl, a hydroxymethyl, a (C₁-C₄)alkoxymethyl, a fluoromethyl, a tetrazolylmethyl, an N-(methyl)tetrazolylmethyl, a tetrazolyl, an N-(methyl)tetrazolyl, a CONR₆R₇ group, a CH₂S(O)ₙAlk group, a COOR₈ group;
- R₄ and R₅ each represent independently a phenyl unsubstituted or substituted with one or more substituents selected independently from a halogen atom, a (C₁-C₇)alkyl, a (C₁-C₄)alkoxy, a trifluoromethyl group or an S(O)ₙAlk group;
- R₆ and R₇ each represent independently a hydrogen atom or a (C₁-C₄)alkyl or R₆ and R₇ together with the nitrogen atom to which they are bound constitute a heterocyclic radical selected from pyrrolidinyl, piperidinyl, morpholinyl, piperazinyl unsubstituted or substituted one or more times with a (C₁-C₄)alkyl;
- R₈ represents a (C₁-C₄)alkyl;
- n represents 0, 1 or 2;
- Alk represents a (C₁-C₄)alkyl;
in the form of base or of salts of addition to acids, as well as in the form of hydrates or of solvates.

2. Compound according to Claim 1 of formula I_{A} in which R₃ is a cyano and the substituents R₁, R₂, R₄, R₅ are as defined for the compounds of formula (I) in Claim 1.

3. Compound according to Claim 1 of formula I_{B} in which R₃ is a hydroxyl and the substituents R₁, R₂, R₄, R₅ are as defined for the compounds of formula (I) in Claim 1.

4. Compound according to Claim 1 of formula I_{C} in which R₃ is a (C₁-C₄)alkoxy and the substituents R₁, R₂, R₄, R₅ are as defined for the compounds of formula (I) in Claim 1.

5. Compound according to Claim 1 of formula IF in which R₃ is a (C₁-C₄)alkoxymethyl and the substituents R₁, R₂, R₄, R₅ are as defined for the compounds of formula (I) in Claim 1.

6. Compound according to Claim 1 of formula I_{I} in which R₃ is a CONR₆R₇ group and the substituents R₁, R₂, R₄, R₅, R₆ and R₇ are as defined for the compounds of formula (I) in Claim 1.

7. Compound according to Claim 1 of formula I_{J} in which R₃ is a COOR₈ and the substituents R₁, R₂, R₄, R₅ and R₈ are as defined for the compounds of formula (I) in Claim 1.

8. Compound according to Claim 1 of formula I_{O} in which R₃ is a tetrazol-1-ylmethyl or a tetrazol-2-ylmethyl and the substituents R₁, R₂, R₄, R₅ are as defined for the compounds of formula (I) in Claim 1.

9. Compound according to Claim 1 of formula I in which:
- R₁ represents:
. a phenyl, benzyl radical, in which each phenyl group is unsubstituted or substituted one or more times with substituents selected independently from a halogen atom, a (C₁-C₄)alkyl, a (C₁-C₄)alkoxy, a trifluoromethyl;
. a furyl radical unsubstituted or substituted with one or more substituents selected independently from a halogen atom, a (C₁-C₄)alkyl, a trifluoromethyl group;
- R₂ represents a hydrogen atom;
- R₃ represents a cyano, a hydroxyl, a (C₁-C₄)alkoxy, a hydroxymethyl, a (C₁-C₄)alkoxymethyl, a CONR₆R₇ group, a COOR₈ group, a tetrazol-1-yl methyl or a tetrazol-2-ylmethyl, the groups R₆, R₇, R₈ being as defined for (I) ;
- R₄ represents a 4-chlorophenyl, a 4-methoxyphenyl;
- R₅ represents a 2-chlorophenyl, a 2-bromophenyl or a 2,4-dichlorophenyl;
in the form of a base or of a salt of addition to acids as well as in the form of hydrates or of solvates.

10. Compound according to Claim 1 of formula I in which:
- R₁ represents a 3-chlorophenyl, 3-fluorophenyl, 3,6-difluorophenyl, 2,6-difluorophenyl, 3-methoxyphenyl, 3-trifluoromethylphenyl, 3-trifluoromethoxyphenyl, a benzyl, a 4-trifluoromethylbenzyl or a 2-trifluoromethyl-4-methylfuryl group;
- R₂ represents a hydrogen atom;
- R₃ represents a cyano, methoxy or dimethylaminocarbonyl group;
- R₄ represents a 4-chlorophenyl, a 4-methoxyphenyl;
- R₅ represents a 2,4-dichlorophenyl or a 2-chlorophenyl;
in the form of a base or of a salt of addition to acids as well as in the form of hydrates or of solvates.

11. Compound according to Claim 1, selected from:
- N-{[5-(4-chlorophenyl)-4-cyano-1-(2,4-dichlorophenyl)-1H-pyrazol-3-yl]methyl}-3-cyanobenzenesulphonamide,
- N-{[5-(4-chlorophenyl)-4-cyano-1-(2,4-dichlorophenyl)-1H-pyrazol-3-yl]methyl}-3-trifluorobenzenesulphonamide,
- N-{[5-(4-chlorophenyl)-4-cyano-1-(2,4-dichlorophenyl)-1H-pyrazol-3-yl]methyl}-2-trifluoromethoxybenzenesulphonamide,
- N-{[5-(4-chlorophenyl)-4-cyano-1-(2,4-dichlorophenyl)-1H-pyrazol-3-yl]methyl}-3-methoxybenzenesulphonamide,
- N-{[5-(4-chlorophenyl)-4-cyano-1-(2,4-dichlorophenyl)-1H-pyrazol-3-yl]methyl}-3-chlorobenzenesulphonamide,
- N-{[5-(4-chlorophenyl)-4-cyano-1-(2-chlorophenyl)-1H-pyrazol-3-yl]methyl}-3-fluorobenzenesulphonamide,
- N-{[5-(4-chlorophenyl)-4-cyano-1-(2-chlorophenyl)-1H-pyrazol-3-yl]methyl}-2-fluorobenzenesulphonamide,
- N-{[5-(4-chlorophenyl)-4-cyano-1-(2-chlorophenyl)-1H-pyrazol-3-yl]methyl}-2-trifluoromethoxybenzenesulphonamide,
- N-{[5-(4-methoxyphenyl)-4-cyano-1-(2,4-dichlorophenyl)-1H-pyrazol-3-yl]methyl}-3.5-difluorobenzenesulphonamide,
- N-{[5-(4-chlorophenyl)-4-methoxy-1-(2,4-dichlorophenyl)-1H-pyrazol-3-yl]methyl}-3-chlorobenzenesulphonamide,
- 5-(4-chlorophenyl)-3-({[(3-chlorophenyl)sulphonyl]amino}methyl)-1-(2,4-dichlorophenyl)-N-methyl-1H-pyrazole-4-carboxamide,
- N-{[5-(4-chlorophenyl)-4-cyano-1-(2,4-dichlorophenyl)-1H-pyrazol-3-yl]methyl}-1-phenylmethanesulphonamide,
- N-{[5-(4-chlorophenyl)-4-cyano-1-(2,4-dichlorophenyl)-1H-pyrazol-3-yl]methyl}-5-methyl-2-(trifluoromethyl)furan-3-sulphonamide;
in the form of a base or of a salt of addition to acids as well as in the form of hydrates or of solvates.

12. Method of preparation of a compound of formula (I) according to Claim 1, **characterized in that** a compound of formula: 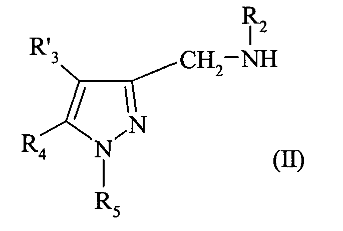 in which R₂, R₄, R₅ are as defined for a compound of formula (I) and R'₃ represents R₃ or a precursor of R₃, is reacted, in the presence of a base, in a solvent, with a sulphonyl halide of formula HalSO₂R₁, in which R₁ is as defined for a compound of formula (I) in Claim 1 and Hal represents a halogen atom; and if applicable, the compound obtained of formula: 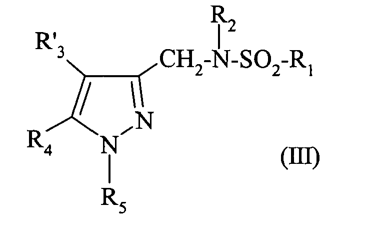 in which R'₃ is R₃ or is a precursor of R₃, is converted to a compound of formula (I).

13. Medicinal product, **characterized in that** it contains a compound of formula (I) according to any one of the Claims 1 to 11, or a hydrate or a solvate of a compound of formula (I).

14. Pharmaceutical composition, **characterized in that** it contains a compound of formula (I) according to any one of the Claims 1 to 11, or a hydrate or a solvate of this compound, as well as at least one pharmaceutically acceptable excipient.

15. Compound of formula (I) as defined in Claims 1 to 11, for the treatment or the prevention of diseases in which the CB₁ receptors are involved.

16. Compound according to Claim 15, **characterized in that** the diseases are psychiatric disorders, substance dependence and withdrawal, cognitive disorders, disorders of attention and vigilance, and acute and chronic neurodegenerative diseases.

17. Compound according to Claim 15, **characterized in that** the diseases are metabolic disorders, disorders of appetency, disorders of appetite, obesity, type II diabetes, metabolic syndrome, and dyslipidaemia.

18. Compound according to Claim 15, **characterized in that** the diseases are pain, neuropathic pain, and pain induced by anticancer treatment.

19. Compound according to Claim 15, **characterized in that** the diseases are gastrointestinal disorders, vomiting, diarrhoea, ulcers, and liver diseases.

20. Compound according to Claim 15, **characterized in that** the diseases are diseases of the immune system, rheumatoid arthritis, demyelinization, multiple sclerosis, and inflammatory diseases.

21. Compound according to Claim 15, **characterized in that** the diseases are Alzheimer's, Parkinson's, schizophrenia, cognitive disorders, diabetes, obesity, metabolic syndrome and tobacco withdrawal.

22. Use of a compound according to any one of Claims 1 to 11, for the preparation of a medicinal product for the treatment or the prevention of psychiatric disorders, substance dependence and withdrawal, cognitive disorders, disorders of attention and vigilance, acute and chronic neurodegenerative diseases, metabolic disorders, disorders of appetency, disorders of appetite, obesity, type II diabetes, metabolic syndrome, dyslipidaemia, pain, neuropathic pain, pain induced by anticancer treatment, gastrointestinal disorders, vomiting, diarrhoea, ulcers, liver diseases, diseases of the immune system, rheumatoid arthritis, demyelinization, multiple sclerosis, inflammatory diseases, Alzheimer's disease, Parkinson's disease, schizophrenia, cognitive disorders, diabetes, obesity, metabolic syndrome and tobacco withdrawal.

## Patentansprüche

1. Verbindung der Formel (I): 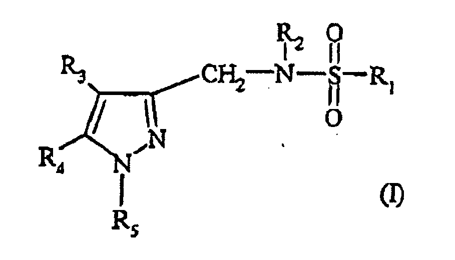 worin:
- R₁ für
. Phenyl, das gegebenenfalls durch einen oder mehrere unabhängig voneinander unter einem Halogenatom, (C₁-C₄)-Alkyl, (C₁-C₄)-Alkoxy, Cyano, einer Trifluormethylgruppe, einer Trifluormethoxygruppe, einer S(O)ₙAlk-Gruppe, einer (C₁-C₄)-Alkylcarbonylgruppe und Phenyl ausgewählte Substituenten substituiert ist;
. Benzyl, das gegebenenfalls durch einen oder mehrere unabhängig voneinander unter einem Halogenatom, (C₁-C₄)-Alkyl, (C₁-C₄)-Alkoxy, und einer Trifluormethylgruppe ausgewählte Substituenten substituiert ist;
. einen Thienyl-, Furyl-, Oxazolyl-, Thiazolyl- oder Imidazolylrest, der gegebenenfalls durch einen oder mehrere unabhängig voneinander unter einem Halogenatom, (C₁-C₄)-Alkyl und einer Trifluormethylgruppe ausgewählte Substituenten substituiert ist;
. Naphthyl, das gegebenenfalls durch einen oder mehrere unabhängig voneinander unter (C₁-C₄)-Alkyl und Di(C₁-C₄)-alkylamino ausgewählte Substituenten substituiert ist;
. 2,3-Dihydrobenzofuranyl, das gegebenenfalls ein- oder mehrfach durch eine (C₁-C₄)-Alkylgruppe substituiert ist;
steht;
- R₂ für ein Wasserstoffatom oder (C₁-C₄)-Alkyl steht;
- R₃ für Cyano, Hydroxyl, (C₁-C₄)-Alkoxy, Cyanomethyl, Hydroxymethyl, (C₁-C₄)-Alkoxymethyl, Fluormethyl, Tetrazolylmethyl, N-(Methyl)-tetrazolylmethyl, Tetrazolyl, N-(Methyl)-tetrazolyl, eine CONR₆R₇-Gruppe, eine CH₂S(O)ₙ-Alkylgruppe oder eine COOR₈-Gruppe steht;
- R₄ und R₅ jeweils unabhängig voneinander für Phenyl, das gegebenenfalls durch einen oder mehrere unabhängig voneinander unter einem Halogenatom, (C₁-C₇)-Alkyl, (C₁-C₄)-Alkoxy, einer Trifluormethylgruppe und einer S(O)ₙAlk-Gruppe ausgewählte Substituenten substituiert ist, stehen;
- R₆ und R₇ jeweils unabhängig voneinander für ein Wasserstoffatom oder (C₁-C₄)-Alkyl stehen oder R₆ und R₇ zusammen mit dem Stickstoffatom, an das sie gebunden sind, einen unter Pyrrolidinyl, Piperidinyl, Morpholinyl und Piperazinyl ausgewählten heterocyclischen Rest, der gegebenenfalls ein- oder mehrfach durch (C₁-C₄)-Alkyl substituiert ist, bilden;
- R₈ für (C₁-C₄)-Alkyl steht;
- n für 0, 1 oder 2 steht;
- Alk für (C₁-C₄)-Alkyl steht;
in Basen- oder Säureadditionssalzform sowie in Hydrat- oder Solvatform.

2. Verbindung nach Anspruch 1 der Formel I_{A}, worin R₃ für Cyano steht und die Substituenten R₁, R₂, R₄ und R₅ wie für die Verbindungen der Formel (I) in Anspruch 1 definiert sind.

3. Verbindung nach Anspruch 1 der Formel I_{B}, worin R₃ für Hydroxyl steht und die Substituenten R₁, R₂, R₄ und R₅ wie für die Verbindungen der Formel (I) in Anspruch 1 definiert sind.

4. Verbindung nach Anspruch 1 der Formel I_{C}, worin R₃ für (C₁-C₄)-Alkoxy steht und die Substituenten R₁, R₂, R₄ und R₅ wie für die Verbindungen der Formel (I) in Anspruch 1 definiert sind.

5. Verbindung nach Anspruch 1 der Formel I_{F}, worin R₃ für (C₁-C₄)-Alkoxymethyl steht und die Substituenten R₁, R₂, R₄ und R₅ wie für die Verbindungen der Formel (I) in Anspruch 1 definiert sind.

6. Verbindung nach Anspruch 1 der Formel I_{I}, worin R₃ für eine CONR₆R₇-Gruppe steht und die Substituenten R₁, R₂, R₄, R₅, R₆ und R₇ wie für die Verbindungen der Formel (I) in Anspruch 1 definiert sind.

7. Verbindung nach Anspruch 1 der Formel I_{J}, worin R₃ für COOR₈ steht und die Substituenten R₁, R₂, R₄, R₅ und R₈ wie für die Verbindungen der Formel (I) in Anspruch 1 definiert sind.

8. Verbindung nach Anspruch 1 der Formel I_{O}, worin R₃ für Tetrazol-1-ylmethyl oder Tetrazol-2-ylmethyl steht und die Substituenten R₁, R₂, R₄ und R₅ wie für die Verbindungen der Formel (I) in Anspruch 1 definiert sind.

9. Verbindung nach Anspruch 1 der Formel I, worin:
- R₁ für:
. einen Phenyl- oder Benzylrest, worin jede Phenylgruppe gegebenenfalls ein- oder mehrfach durch unabhängig voneinander unter einem Halogenatom, (C₁-C₄)-Alkyl, (C₁-C₄)-Alkoxy und Trifluormethyl ausgewählte Substituenten substituiert ist;
. einen Furylrest, der gegebenenfalls durch einen oder mehrere unabhängig voneinander unter einem Halogenatom, (C₁-C₄)-Alkyl und einer Trifluormethylgruppe ausgewählte Substituenten substituiert ist;
steht;
- R₂ für ein Wasserstoffatom steht;
- R₃ für Cyano, Hydroxyl, (C₁-C₄)-Alkoxy, Hydroxymethyl, (C₁-C₄)-Alkoxymethyl, eine CONR₆R₇-Gruppe, eine COOR₈-Gruppe, Tetrazol-1-ylmethyl oder Tetrazol-2-ylmethyl steht, wobei die Substituenten R₆, R₇ und R₈ wie für (I) definiert sind;
- R₄ für 4-Chlorphenyl oder 4-Methoxyphenyl steht;
- R₅ für 2-Chlorphenyl, 2-Bromphenyl oder 2,4-Dichlorphenyl steht;
in Basen- oder Säureadditionssalzform sowie in Hydrat- oder Solvatform.

10. Verbindung nach Anspruch 1 der Formel I, worin:
- R₁ für eine 3-Chlorphenyl-, 3-Fluorphenyl-, 3,6-Difluorphenyl-, 2,6-Difluorphenyl-, 3-Methoxyphenyl-, 3-Trifluormethylphenyl-, 3-Trifluormethoxyphenyl-, Benzyl-, 4-Trifluormethylbenzyl- oder 2-Trifluormethyl-4-methylfurylgruppe steht;
- R₂ für ein Wasserstoffatom steht;
- R₃ für eine Cyano-, Methoxy- oder Dimethylaminocarbonylgruppe steht;
- R₄ für 4-Chlorphenyl oder 4-Methoxyphenyl steht;
- R₅ für 2,4-Dichlorphenyl oder 2-Chlorphenyl steht;
in Basen- oder Säureadditionssalzform sowie in Hydrat- oder Solvatform.

11. Verbindung nach Anspruch 1, ausgewählt unter:
- N-{[5-(4-Chlorphenyl)-4-cyano-1-(2,4-dichlorphenyl)-1*H*-pyrazol-3-yl]methyl}-3-cyanobenzolsulfonamid,
- N-{[5-(4-Chlorphenyl)-4-cyano-1-(2,4-dichlorphenyl)-1*H*-pyrazol-3-yl]methyl}-3-trifluorbenzolsulfonamid,
- N-{[5-(4-Chlorphenyl)-4-cyano-1-(2,4-dichlorphenyl)-1*H*-pyrazol-3-yl]methyl}-2-trifluormethoxybenzolsulfonamid,
- N-{[5-(4-Chlorphenyl)-4-cyano-1-(2,4-dichlorphenyl)-1*H*-pyrazol-3-yl]methyl}-3-methoxybenzolsulfonamid,
- N-{[5-(4-Chlorphenyl)-4-cyano-1-(2,4-dichlorphenyl)-1*H*-pyrazol-3-yl]methyl}-3-chlorbenzolsulfonamid,
- N-{[5-(4-Chlorphenyl)-4-cyano-1-(2-chlorphenyl)-1*H*-pyrazol-3-yl]methyl}-3-fluorbenzolsulfonamid,
- N-{[5-(4-Chlorphenyl)-4-cyano-1-(2-chlorphenyl)-1*H*-pyrazol-3-yl]methyl}-2-fluorbenzolsulfonamid,
- N-{[5-(4-Chlorphenyl)-4-cyano-1-(2-chlorphenyl)-1*H*-pyrazol-3-yl]methyl}-2-trifluormethoxybenzolsulfonamid,
- N-{[5-(4-Methoxyphenyl)-4-cyano-1-(2,4-dichlorphenyl)-1*H*-pyrazol-3-yl]methyl}-3,5-difluorbenzolsulfonamid,
- N-{[5-(4-Chlorphenyl)-4-methoxy-1-(2,4-dichlorphenyl)-1*H*-pyrazol-3-yl]methyl}-3-chlorbenzolsulfonamid,
- 5-(4-Chlorphenyl)-3-({[(3-chlorphenyl)sulfonyl]-amino}methyl)-1-(2,4-dichlorphenyl)-N-methyl-1*H-*pyrazol-4-carboxamid,
- N-{[5-(4-Chlorphenyl)-4-cyano-1-(2,4-dichlorphenyl)-1*H*-pyrazol-3-yl]methyl}-1-phenylmethansulfonamid,
- N-{[5-(4-Chlorphenyl)-4-cyano-1-(2,4-dichlorphenyl)-1*H*-pyrazol-3-yl]methyl}-5-methyl-2-(trifluormethyl)furan-3-sulfonamid;
in Basen- oder Säureadditionssalzform sowie in Hydrat- oder Solvatform.

12. Verfahren zur Herstellung einer Verbindung der Formel (I) nach Anspruch 1, **dadurch gekennzeichnet, daß** man eine Verbindung der Formel: 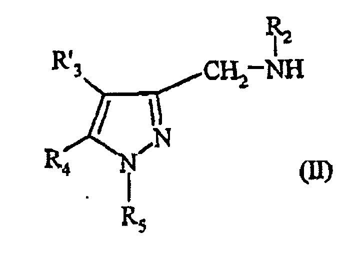 worin R₂, R₄ und R₅ wie für eine Verbindung der Formel (I) definiert sind und R'₃ für R₃ oder eine Vorstufe von R₃ steht, in Gegenwart einer Base in einem Lösungsmittel mit einem Sulfonylhalogenid der Formel HalSO₂R₁, worin R₁ wie für eine Verbindung der Formel (I) in Anspruch 1 definiert ist und Hal für ein Halogenatom steht, umsetzt und gegebenenfalls die erhaltene Verbindung der Formel: 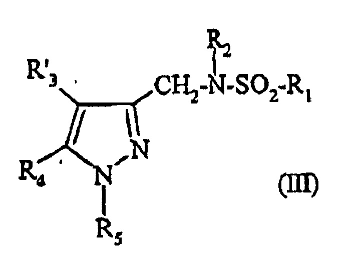 worin R'₃ für R₃ oder eine Vorstufe von R₃ steht, in eine Verbindung der Formel (I) umwandelt.

13. Arzneimittel, **dadurch gekennzeichnet, daß** es eine Verbindung der Formel (I) nach einem der Ansprüche 1 bis 11 oder ein Hydrat oder ein Solvat einer Verbindung der Formel (I) enthält.

14. Pharmazeutische Zusammensetzung, **dadurch gekennzeichnet, daß** sie eine Verbindung der Formel (I) nach einem der Ansprüche 1 bis 11 oder ein Hydrat oder ein Solvat der Verbindung der Formel (I) sowie einen pharmazeutisch unbedenklichen Hilfsstoff enthält.

15. Verbindung der Formel (I) gemäß den Ansprüchen 1 bis 11 zur Behandlung oder Prävention von Erkrankungen, an denen die CB₁-Rezeptoren beteiligt sind.

16. Verbindung nach Anspruch 15, **dadurch gekennzeichnet, daß** es sich bei den Erkrankungen um psychiatrische Störungen, Substanzabhängigkeit und -entwöhnung, kognitive Störungen, Aufmerksamkeits- und Wachsamkeitsstörungen und akute und chronische neurodegenerative Erkrankungen handelt.

17. Verbindung nach Anspruch 15, **dadurch gekennzeichnet, daß** es sich bei den Erkrankungen um Stoffwechselstörungen, Appetenzstörungen, Appetitstörungen, Obesitas, Typ-II-Diabetes, metabolisches Syndrom und Dyslipidämie handelt.

18. Verbindung nach Anspruch 15, **dadurch gekennzeichnet, daß** es sich bei den Erkrankungen um Schmerzen, neuropathische Schmerzen und durch Antikrebsbehandlung induzierte Schmerzen handelt.

19. Verbindung nach Anspruch 15, **dadurch gekennzeichnet, daß** es sich bei den Erkrankungen um Magen-Darm-Störungen, Erbrechen, Durchfallstörungen, Geschwüre und Lebererkrankungen handelt.

20. Verbindung nach Anspruch 15, **dadurch gekennzeichnet, daß** es sich bei den Erkrankungen um Erkrankungen des Immunsystems, rheumatoide Arthritis, Demyelinisierung, multiple Sklerose und entzündliche Erkrankungen handelt.

21. Verbindung nach Anspruch 15, **dadurch gekennzeichnet, daß** es sich bei den Erkrankungen um Alzheimer-Krankheit, Parkinson-Krankheit, Schizophrenie, kognitive Störungen, Diabetes, Obesitas, metabolisches Syndrom und Tabakentwöhnung handelt.

22. Verwendung einer Verbindung nach einem der Ansprüche 1 bis 11 zur Herstellung eines Arzneimittels zur Behandlung oder Prävention von psychiatrischen Störungen, Substanzabhängigkeit und -entwöhnung, kognitiven Störungen, Aufmerksamkeits- und Wachsamkeitsstörungen, akuten und chronischen neurodegenerativen Erkrankungen, Stoffwechselstörungen, Appetenzstörungen, Appetitstörungen, Obesitas, Typ-II-Diabetes, metabolischem Syndrom, Dyslipidämie, Schmerzen, neuropathischen Schmerzen, durch Antikrebsbehandlung induzierten Schmerzen, Erkrankungen um Magen-Darm-Störungen, Erbrechen, Durchfallstörungen, Geschwüren, Lebererkrankungen, Erkrankungen des Immunsystems, rheumatoider Arthritis, Demyelinisierung, multipler Sklerose, entzündlichen Erkrankungen, Alzheimer-Krankheit, Parkinson-Krankheit, Schizophrenie, kognitiven Störungen, Diabetes, Obesitas, metabolischem Syndrom und Tabakentwöhnung.
